(19) Europäisches Patentamt
European Patent Office
Office européen des brevets

(11) EP 4 640 672 A1

(12) **EUROPEAN PATENT APPLICATION**
published in accordance with Art. 153(4) EPC

(43) Date of publication:
**29.10.2025 Bulletin 2025/44**

(21) Application number: **22968979.9**

(22) Date of filing: **23.12.2022**

(51) International Patent Classification (IPC):
***C07D 211/58*** (2006.01) ***C07D 401/12*** (2006.01)
***C07D 405/12*** (2006.01) ***A61K 31/4468*** (2006.01)
***A61K 31/4545*** (2006.01) ***A61K 31/453*** (2006.01)
***A61P 35/00*** (2006.01)

(52) Cooperative Patent Classification (CPC):
**A61K 31/4468; A61K 31/453; A61K 31/4545; A61P 35/00; C07D 211/58; C07D 401/12; C07D 405/12**

(86) International application number:
**PCT/CN2022/141338**

(87) International publication number:
**WO 2024/130690 (27.06.2024 Gazette 2024/26)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Jiangsu Protelight Pharmaceutical & Biotechnology Co., Ltd.**
**Jiangyin, Jiangsu 214429 (CN)**

(72) Inventors:
- **CHEN, Yuxin**
  **Jiangyin, Jiangsu 214429 (CN)**
- **CHEN, Mingxia**
  **Jiangyin, Jiangsu 214429 (CN)**
- **YAO, Wenjun**
  **Jiangyin, Jiangsu 214429 (CN)**

(74) Representative: **CMS Cameron McKenna Nabarro Olswang LLP**
**Cannon Place**
**78 Cannon Street**
**London EC4N 6AF (GB)**

(54) **ETHYLENEDIAMINE DERIVATIVE, AND PREPARATION METHOD THEREFOR AND USE THEREOF**

(57) Disclosed in the present application are an ethylenediamine derivative, and a preparation method therefor and a use thereof. The structural formula of the ethylenediamine derivative is represented by Formula I. The compound can be prepared by an artificial synthesis method, has a broad-spectrum anti-tumor effect, can prolong the survival period of a tumor patient, and improves the quality of life of the tumor patient. The compound has a stable drug effect and low toxicity, is easily accepted by a human body, can be applied to the treatment of most cancers, and has certain advantages compared with currently listed anti-tumor drugs.

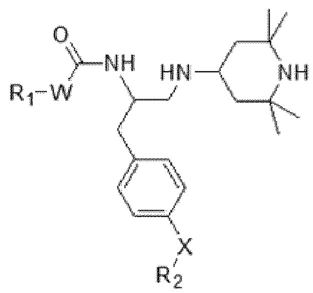

Formula I

EP 4 640 672 A1

## Description

### Technical Field

The present application is in the field of medicine , and particularly relates to an ethylenediamine derivative and preparation method therefor and use thereof.

### Background Art

Bradykinin (BK) has been found to be an important growth factor in many cancers, which can promote neovascularization by stimulating the secretion of vascular endothelial growth factor, and can also stimulate cancer cell migration and invasion by activating matrix metalloproteinase (MMP) active enzymes.

The effect of BK on the body is exerted by binding to the B1 receptor (B1R) and B2 receptor (B2R) on the cell membrane. Both B1R and B2R are G protein-coupled receptors. Research findings on cancer: B1R not only up-regulated abnormally in malignant prostate tumors, but also stimulated the activation of macrophages and dendritic cells in the tumor microenvironment; B2R is not only overexpressed in human brain gliomas, but has also been detected in gastric, duodenal, prostate, lung, and liver cancers.

BK, whether it binds to B1 or B2 receptors, mainly stimulates phospholipase C-β (PLC-β) through Gq subunit in the G protein-coupled receptor family, promotes inositol trisphosphate (IP3) hydrolysis and intracellular $Ca^{2+}$ mobilization to generate NO, and inhibits adenylate cyclase (AC) through G protein subunit Gα-i to activate the mitogen-activated protein kinase pathway (MAPK) to promote cancer cell proliferation, migration and invasion. Among them, the MAPK pathway involves P38, proto-oncogene N-terminal kinase (JNK), and ERK.

Based on this target, a series of inhibitors have been developed, such as compounds PL-AC-15, PL-AC-202, etc. Among them, PL-AC-15 is an amino acid derivative developed by Jiangsu ProteLight Pharmaceutical & Biotechnology Co., Ltd. (see Chinese patent CN107382827B), which has a good anti-tumor effect. PL-AC-202 is a compound obtained by Jiangsu ProteLight Pharmaceutical & Biotechnology Co., Ltd. through further structural optimization and modification on the basis of PL-AC-15 (see Chinese Patent Application No. 202010386293.7), with a view to obtaining a compound with better anti-tumor effect through further structural modification.

### Summary of the Invention

One object of the present application is to provide a class of ethylenediamine derivatives and pharmaceutically acceptable salts, esters, solvates, or isomers thereof, including stereoisomers, enantiomers, tautomers, or mixtures thereof.

The structural formula of ethylenediamine derivatives provided by the present application is represented by the following Formula I:

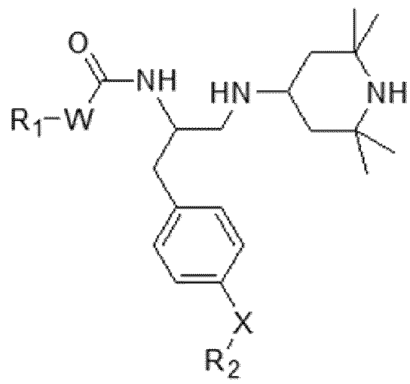

Formula I

wherein, $R_1$ is selected from any one of the following groups: $C_{1-10}$ alkyl, $C_{2-8}$ alkenyl, $C_{2-8}$ alkynyl, $C_{3-12}$ cycloalkyl, 3-12 membered heterocyclyl, $C_{6-14}$ aryl, oxy $C_{6-14}$ aryl, or oxy $C_{5-14}$ arheteryl, nitrogen-based $C_{6-14}$ aryl, or nitrogen-based $C_{5-14}$ arheteryl, 5-14 membered heteroaryl. The hydrogen on $R_1$ may be optionally substituted by one or more of the following substituents: halogen, $C_{1-10}$ alkyl, $C_{2-8}$ alkenyl, $C_{2-8}$ alkynyl, $C_{3-12}$ cycloalkyl, 3-12 membered heterocyclyl, $C_{6-14}$ aryl, oxy $C_{6-14}$ aryl or oxy $C_{5-14}$ arylheteryl, nitrogen-based $C_{6-14}$ aryl or nitrogen-based $C_{5-14}$ arylheteryl, 5-14 membered heteroaryl, -CN, $-NO_2$, $-CF_2H$, $-CF_2OH$, $-CF_3$, $-OCF_3$, $-CR^1R^2R^3$, $-OR^1$, $-O(C=O)R^1$,

-O(C=O)$OR^1$, -O(C=O)$NR^2R^3$, -(C=O)$R^1$, -(C=O)$OR^1$, -(C=O)$NR^2R^3$, -$SR^1$, -(S=O)$_mR^1$, -$NR^2R^3$, -$NR^4$(C=O)$R^1$, -$NR^4$C(=O)$NR^2R^3$, -$NR^4$C(=O)$OR^1$, -$NR^4$S(=O)$_mNR^2R^3$, -$NR^4$S(=O)$_mOR^1$ or -$NR^4$S(=O)$_mR^1$, or the groups of adjacent atoms on $R_1$ may combine to form $C_{3-12}$ cycloalkyl, $C_{6-12}$ aryl, 3-12 membered heterocyclic and 5-12 membered heteroaryl ring groups;

wherein $R^1$, $R^2$, $R^3$, and $R^4$ may be independently selected from hydrogen, halogen or anyof the following groups: $C_{1-10}$ alkyl, $C_{2-8}$ alkenyl, $C_{2-8}$ alkynyl, $C_{3-12}$ cycloalkyl, 3-12 membered heterocyclyl, $C_{6-14}$ aryl, oxy $C_{6-14}$ aryl, or oxy $C_{5-14}$ arheteryl, nitrogen-based $C_{6-14}$ aryl, or nitrogen-based $C_{5-14}$ arheteryl, 5-14 membered heteroaryl, or any two of $R^1$, $R^2$, $R^3$, and $R^4$ bound to the same nitrogen atom may be combined with the nitrogen to which they are bound to form a 3-12 membered heterocyclyl or 5-12 membered heteroaryl, which optionally containing 1 to 3 additional heteroatoms selected from N, O, and S, or any two of $R^1$, $R^2$, and $R^3$ bound to the same carbon atom may combine to form a $C_{3-12}$ cycloalkyl, $C_{6-12}$ aryl, 3-12 membered heterocyclyl, or 5-12 membered heteroaryl; and each hydrogen in $R^1$, $R^2$, $R^3$, and $R^4$ is optionally substituted with $R^5$, or two hydrogen atoms on the same carbon atom in $R^1$, $R^2$, $R^3$, and $R^4$ are optionally oxo substituents.

$R^5$ may be independently selected from the group consisting of: hydrogen, halogen, $C_{1-10}$ alkyl, $C_{2-8}$ alkenyl, $C_{2-8}$ alkynyl, $C_{3-12}$ cycloalkyl, 3-12 membered heterocyclyl, $C_{6-14}$ aryl, oxy $C_{6-14}$ aryl, or oxy $C_{5-14}$ arheteryl, nitrogen-based $C_{6-14}$ aryl, or nitrogen-based $C_{5-14}$ arheteryl, 5-14 membered heteroaryl; -CN, -$NO_2$, -OH, -$NH_2$, partially or fully halogenated $C_{1-5}$ alkyl, -C(= O)$(CH_2)_nCH_3$, -C( = O)O$(CH_2)_nCH_3$, -C(=O)OH, -C( = O)N$[(CH_2)_nCH_3]_2$, -C(=O)$NH_2$, -C(=O)NH$(CH_2)_nCH_3$, -NH$(CH_2)_nCH_3$, -N$[(CH_2)_nCH_3]_2$, -N$(CH_2)_nCH_3$C(=O)$(CH_2)_nCH_3$, -N$(CH_2)_nCH_3$C(=O)NH$(CH_2)_nCH_3$, -N$(CH_2)_nCH_3$C(=O)N$[(CH_2)_nCH_3]_2$, -N$(CH_2)_nCH_3$C(=O)$NH_2$, -N$(CH_2)_nCH_3$C(=O)O$(CH_2)_nCH_3$, -N$(CH_2)_nCH_3$C(=O)OH, -NHC(=O)$(CH_2)_nCH_3$, -NHC(=O)NH$(CH_2)_nCH_3$, -NHC(=O)N$[(CH_2)_nCH_3]_2$, -NHC(=O)$NH_2$, -NHC(=O)O$(CH_2)_nCH_3$, -NHC(=O)OH, -N$(CH_2)_nCH_3$S(=O)$_m(CH_2)_nCH_3$, -NHS(=O)$_m(CH_2)_nCH_3$, -O$(CH_2)_nCH_3$, =O, -OC(=O)$(CH_2)_nCH_3$, OC(=O)O$(CH_2)_nCH_3$, -OC(=O)N$[(CH_2)_nCH_3]_2$, -OC(=O)NH$(CH_2)_nCH_3$, -OC(=O)$NH_2$, -S(=O)$_m$(CH2)$_nCH_3$, -OS(=O)$_m$(CH2)$_nCH_3$, -S(=O)$_m$NH$(CH_2)_nCH_3$, -S(= O)$_m$N$[(CH_2)_nCH_3]_2$;

the m is selected from 1 or 2;

the n is selected from 1, 2, 3, 4 or 5.

$R_2$ is selected from any one of the following groups: H, halogen, $C_{1-10}$ alkyl, oxy $C_{1-8}$ alkyl, $C_{2-8}$ alkenyl, $C_{2-8}$ alkynyl, $C_{3-12}$ cycloalkyl, 3-12 membered heterocyclyl, $C_{6-14}$ aryl, oxy $C_{6-14}$ aryl, oxy $C_{5-14}$ arylheteryl, nitrogen-based $C_{6-14}$ aryl, nitrogen-based $C_{5-14}$ arylheteryl, 5-14 membered heteroaryl.

The hydrogen on $R_2$ may be optionally substituted by one or more of the following substituents: halogen, -CN, -$NO_2$, -$CF_2$H, -$CF_2$OH, -$CF_3$, -$OCF_3$, -$CR^6R^7R^8$, -$OR^6$, -O(C=O)$R^6$, -O(C=O)$OR^6$, -O(C=O)$NR^7R^8$, -(C=O)$R^6$, -(C=O)$OR^6$, -(C=O)$NR^7R^8$, -$SR^6$, -(S=O)$R^6$, -S(=O)$_2R^6$, -$NR^7R^8$, -$NR^9$(C=O)$R^6$, -$NR^9$C(=O)$NR^7R^8$, -$NR^9$C(=O)$OR^6$, -$NR^9$S(=O)$_mNR^7R^8$, -$NR^9$S(=O)$_mOR^6$ or -$NR^9$S(=O)$_mR^6$, or groups of adjacent atoms on $R_2$ may combine to form $C_{3-12}$ cycloalkyl, $C_{6-12}$ aryl, 3-12 membered heterocyclic, and 5-12 membered heteroaromatic ring;

wherein $R^6$, $R^7$, $R^8$, and $R^9$ may be hydrogen or any one selected from $C_{1-10}$ alkyl, $C_{2-8}$ alkenyl, $C_{2-8}$ alkynyl, $C_{3-12}$ cycloalkyl, 3-12 membered heterocyclyl, $C_{6-14}$ aryl, oxy $C_{6-14}$ aryl, or oxy $C_{5-14}$ arheteryl, nitrogen-based $C_{6-14}$ aryl, or nitrogen-based $C_{5-14}$ arheteryl, 5-14 membered heteroaryl, or any two of R1, R2, R3, and R4 bound to the same nitrogen atom may be combined with the nitrogen to which they are bound to form a 3-12 membered heterocyclic or 5-12 membered heteroaryl, which optionally containing 1 to 3 additional heteroatoms selected from N, O and S; or any two of $R^6$, $R^7$, $R^8$, and $R^9$ bound to the same carbon atom may combine to form a $C_{3-12}$ cycloalkyl, $C_{6-12}$ aryl, 3-12 membered heterocyclyl, or 5-12 membered heteroaryl; and each hydrogen in $R^6$, $R^7$, and $R^8$ is optionally substituted with $R^{10}$, or two hydrogen atoms on the same carbon atom in $R^6$, $R^7$, $R^8$, and $R^9$ are optionally oxo substituents.

$R^{10}$ may be independently selected from: hydrogen, halogen, $C_{1-10}$ alkyl, $C_{2-8}$ alkenyl, $C_{2-8}$ alkynyl, $C_{3-12}$ cycloalkyl, 3-12 membered heterocyclyl, $C_{6-14}$ aryl, oxy $C_{6-14}$ aryl or oxy $C_{5-14}$ arylheteryl, nitrogen-based $C_{6-14}$ aryl or nitrogen-based $C_{5-14}$ arylheteryl, 5-14 membered heteroaryl, -CN, -$NO_2$, -OH, -$NH_2$, partially or fully halogenated $C_{1-5}$ alkyl, -C(=O)$(CH_2)_nCH_3$, -C(=O)O$(CH_2)_nCH_3$, -C(=O)OH, -C(= O)N$[(CH_2)_nCH_3]_2$, -C(=O)$NH_2$, -C(=O)NH$(CH_2)_nCH_3$, -NH$(CH_2)_nCH_3$, -N$[(CH_2)_nCH_3]_2$, -N$(CH_2)_nCH_3$C(=O)$(CH_2)_nCH_3$, -N$(CH_2)_nCH_3$C(=O)NH$(CH_2)_nCH_3$, -N$(CH_2)_nCH_3$C(=O)N$[(CH_2)_nCH_3]_2$, -N$(CH_2)_nCH_3$C(=O)$NH_2$, -N$(CH_2)_nCH_3$C(=O)O$(CH_2)_nCH_3$, -N$(CH_2)_nCH_3$C(=O)OH, -NHC(=O)$(CH_2)_nCH_3$, -NHC(=O)NH$(CH_2)_nCH_3$, -NHC(=O)N$[(CH_2)_nCH_3]_2$, -NHC(=O)$NH_2$, -NHC(=O)O$(CH_2)_nCH_3$, -NHC(=O)OH, -N$(CH_2)_nCH_3$S(=O)$_m(CH_2)_nCH_3$, -NHS(=O)$_m(CH_2)_nCH_3$, -O$(CH_2)_nCH_3$, =O, -OC(=O)$(CH_2)_nCH_3$, OC(=O)O$(CH_2)_nCH_3$, -OC(=O)N$[(CH_2)_nCH_3]_2$, -OC(=O)NH$(CH_2)_nCH_3$, -OC(=O)$NH_2$, -S(=O)$_m$(CH2)$_nCH_3$, -OS(=O)$_m$(CH2)$_nCH_3$, -S(=O)$_m$NH$(CH_2)_nCH_3$, -S(= O)$_m$N$[(CH_2)_nCH_3]_2$;

the m is selected from 1 or 2;

the n is selected from 1, 2, 3, 4 or 5.

W represents a bond or is selected from any one of the following groups: $C_{1-8}$ alkylene (e.g.-$CH_2CH_2$-), $C_{2-8}$ alkenylene (e.g.-$CH_2$ = CH-$CH_2$-), $C_{2-8}$ alkynylene (e.g.-C≡C-$CH_2$-), $C_{3-8}$ cycloalkylene, 3-8 membered heterocyclylene,

oxy $C_{1-8}$ alkylene, -O-, -NH-, amino $C_{1-8}$ alkylene, or any of the above groups in which one or more hydrogens are substituted with halogens, and any of the above groups in which a hydrogen is substituted with $R^{11}$, wherein $R^{11}$ is defined as for$R^1$.

The $C_{3-8}$ cycloalkylene groups include, but are not limited to, the following groups:

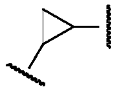 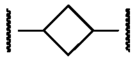 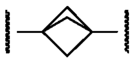 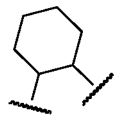

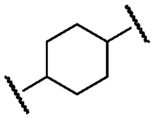 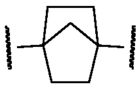 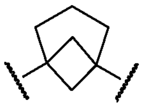 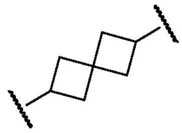

The 3-8 membered heterocyclylene groups include, but are not limited to, the following groups:

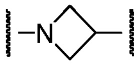 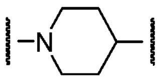 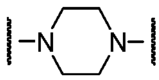 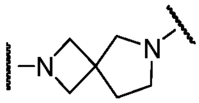

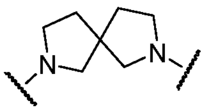 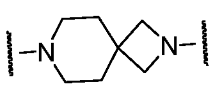 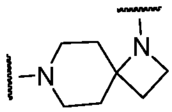

the oxy $C_{1-8}$ alkylene, such as: $-OCH_2-$, $-CH_2O-$,

the amino $C_{1-8}$ alkylene, such as: $-NHCH_2-$, $-CH_2NH-$, $-NCH_3CH_2-$, - $CH_2$ $CH_3N-$,

X represents a chemical bond or is selected from any one of the following groups: $C_{1-8}$ alkylene (e.g.$-CH_2-$, $-CH_2CH_2-$, $-CHCH_3CH_2-$), -O-, oxy $C_{1-8}$ alkylene (e.g. $-OCH_2-$, $-CH_2O-$), -NH-, amino $C_{1-8}$ alkylene (e.g: $-NHCH_2-$, $-CH_2NH-$, $-NCH_3CH_2-$), mercapto $C_{1-8}$ alkylene (e.g: $-SCH_2-$, $-CH_2S-$), oxidized mercapto $C_{1-8}$ alkylene (e.g: $-S(=O)CH_2-$, $-CH_2S(=O)_2-$), -S(= O)NH-, $-S(=O)_2NH-$, or any of the above groups in which one or more of the hydrogens on X is substituted with halo, and any of the above groups in which hydrogen on X is substituted with $R^{12}$. Wherein $R^{12}$ is as defined for $R^1$.

Preferably $R_1$ is selected from any one of the following: $C_{1-4}$ alkyl, $C_{2-4}$ alkenyl, $C_{3-7}$ cycloalkyl, phenyl, phenoxy, fluorenyl, pyridyl, substituted pyridyl, and a group in which hydrogen of $R_1$ is optionally substituted with $R^{13}$, wherein $R^{13}$ is as defined for $R^{10}$;

preferably $R_2$ is selected from any one of the following: H, halogen, phenyl, biphenyl, 5-6 membered heteroaryl, $C_{3-8}$ cycloalkyl, 3-8 membered heterocyclyl, and a group wherein any hydrogen on $R_2$ is substituted with $R^{14}$, wherein $R^{14}$ is as defined for $R^{10}$;

preferably W represents a chemical bond or is selected from any one of the following groups: $C_{1-4}$ alkylene, $C_{2-6}$ alkenylene, $-OCH_2-$, $-CH_2O-$;

preferably X represents a chemical bond or is selected from any one of the following groups: $-CH_2-$, $-CH_2CH_2-$, oxy $C_{1-4}$ alkylene;

further, $R_1$ in combination with the W group in Formula I includes, but is not limited to, the following moieties:

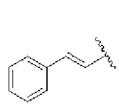 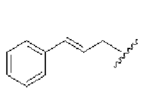 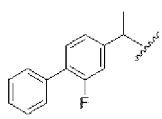 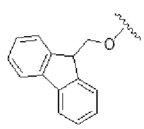

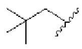 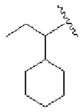 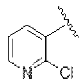 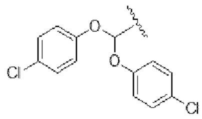

X in Formula I in combination with $R_2$ includes, but is not limited to, the following moieties:

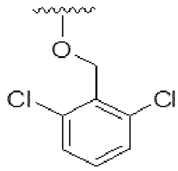 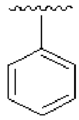 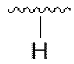 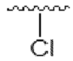

In some of these embodiments, the ethylenediamine derivatives described herein may be exemplified by, but not limited to, the structures shown below (see Table 1 for overall compound structures):

Table 1 List of ethylenediamine derivatives according to the present application

| No. | Structural formula | M+H | $^1$H NMR | Name |
|---|---|---|---|---|
| Cpd 001 | 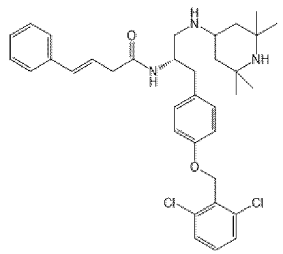 | 608.2 | $^1$H NMR (400 MHz, DMSO-$d_6$) δ 8.12 (s, 2H), 7.74 (d, J = 8.2 Hz, 1H), 7.59 - 7.53 (m, 2H), 7.47 (dd, J = 8.9, 7.1 Hz, 1H), 7.39 (d, J = 7.2 Hz, 2H), 7.36 - 7.28 (m, 2H), 7.26 - 7.16 (m, 1H), 7.19 - 7.12 (m, 2H), 6.96 - 6.88 (m, 2H), 6.44 (d, J = 15.9 Hz, 1H), 6.28 (dt, J = 15.8, 7.0 Hz, 1H), 5.20 - 5.08 (m, 2H), 3.95 (s, 1H), 3.30 (s, 1H), 3.11 (s, 1H), 3.00 (d, J = 7.1 Hz, 2H), 2.94 (s, 1H), 2.78 (dd, J = 13.8, 5.3 Hz, 1H), 2.61 (t, J = 7.0 Hz, 3H), 1.85 (t, J = 16.1 Hz, 2H), 1.34 - 1.21 (m, 13H), 1.13 (d, J = 12.2 Hz, 2H). | (*S*,*E*)-*N*-(1-(4-((2,6-dichlor obenzyl)oxy)phenyl)-3-((2, 2,6,6-tetramethylpiperidin-4-yl)amino)propan-2-yl)-4-phenylbut-3-enamide |
| Cpd 002 | 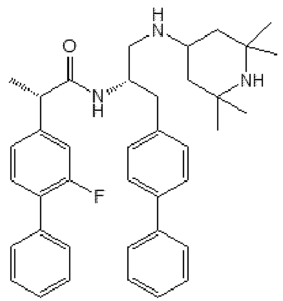 | 592.4 | $^1$H NMR (400 MHz, $CDCl_3$) δ 7.58 - 7.29 (m, 13H), 7.20 (d, *J* = 7.9 Hz, 2H), 7.16 - 7.08 (m, 2H), 6.26 (s, 1H), 4.24 (d, *J* = 7.0 Hz, 1H), 3.61 (q, *J* = 7.1 Hz, 1H), 3.48 (s, 2H), 2.92 (dd, *J* = 13.7, 6.7 Hz, 1H), 2.81 (dd, *J* = 13.8, 7.1 Hz, 1H), 2.69 (s, 1H), 1.78 (d, *J* = 13.1 Hz, 1H), 1.65 (d, *J* = 13.1 Hz, 1H), 1.52 - 1.44 (m, 7H), 1.40 (d, *J* = 8.3 Hz, 6H), 1.33 (s, 3H), 1.25 (t, *J* = 3.5 Hz, 1H), 0.86 (s, 1H), 0.07 (s, 1H). | (*S*)-*N*-((S)-1-([1,1'-bipheny l]-4-yl)-3-((2,2,6,6-tetrame thylpiperidin-4-yl)amino)p ropan-2-yl)-2-(2-fluoro-[1, 1'-biphenyl]-4-yl)propana mide |
| Cpd 003 | 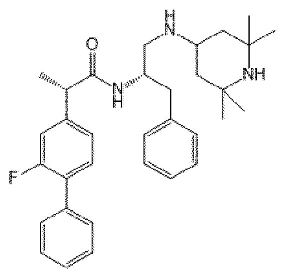 | 516.25 | $^1$H NMR (400 MHz, DMSO-$d_6$) δ 7.83 (d, *J* = 8.4 Hz, 1H), 7.57 - 7.45 (m, 4H), 7.47 - 7.34 (m, 2H), 7.15 - 7.04 (m, 5H), 7.02 (dd, *J* = 7.3, 2.2 Hz, 2H), 3.93 (d, *J* = 6.3 Hz, 1H), 3.62 (q, *J* = 7.0 Hz, 1H), 2.86 - 2.74 (m, 2H), 2.57 (dd, *J* = 13.4, 7.6 Hz, 3H), 1.70 (t, *J* = 13.9 Hz, 2H), 1.32 (d, *J* = 7.0 Hz, 3H), 1.28 - 1.21 (m, 2H), 1.15 (s, 6H), 1.08 (s, 6H), 1.04 (s, 0H), 0.89 - 0.82 (m, 3H). | (*S*)-2-(2-fluoro-[1,1'-biphe nyl]-4-yl)-*N*-((*S*)-1-phenyl-3-((2,2,6,6-tetramethylpipe ridin-4-yl)amino)propan-2-yl)propanamide |

(continued)

| No. | Structural formula | M+H | $^1$H NMR | Name |
|---|---|---|---|---|
| Cpd 004 | 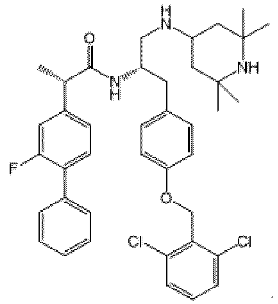 | 690.3 | $^1$H NMR (400 MHz, DMSO-$d_6$) $\delta$ 7.85 (d, J = 8.7 Hz, 1H), 7.60 - 7.49 (m, 4H), 7.53 - 7.42 (m, 4H), 7.46 - 7.35 (m, 1H), 7.27 - 7.19 (m, 2H), 7.18 - 7.11 (m, 2H), 7.01 - 6.92 (m, 2H), 5.18 (s, 2H), 3.95 (d, J = 7.1 Hz, 1H), 3.63 (q, J = 7.0 Hz, 1H), 3.29 (s, 1H), 2.83 (s, 1H), 2.79 (dd, J = 13.7, 5.3 Hz, 1H), 2.66 - 2.56 (m, 2H), 2.53 (s, 1H), 1.82 - 1.73 (m, 1H), 1.58 (d, J = 13.1 Hz, 1H), 1.30 - 1.24 (m, 6H), 1.23 (d, J = 16.0 Hz, 10H), 1.03 (s, 2H). | (*S*)-*N*-((*S*)-1-(4-((2,6-dichl orobenzyl)oxy)phenyl)-3-( (2,2,6,6-tetramethylpiperid in-4-yl)amino)propan-2-yl) -2-(2-fluoro-[1,1'-biphenyl] -4-yl)propanamide |
| Cpd 005 | 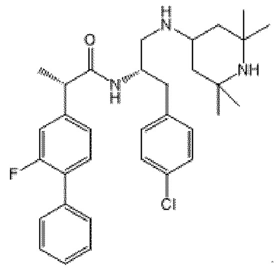 | 550.25 | $^1$H NMR (400 MHz, DMSO-$d_6$) $\delta$ 7.58 - 7.44 (m, 5H), 7.44 - 7.36 (m, 1H), 7.28 - 7.19 (m, 2H), 3.77 (q, J = 7.1 Hz, 1H), 1.41 (d, J = 7.1 Hz, 3H), 1.38 - 1.25 (m, 0H), 1.23 (s, 2H). | (*S*)-*N*-((*S*)-1-(4-chlorophen yl)-3-((2,2,6,6-tetramethyl piperidin-4-yl)amino)propa n-2-yl)-2-(2-fluoro-[1,1'-bi phenyl]-4-yl)propanamide |
| Cpd 006 | 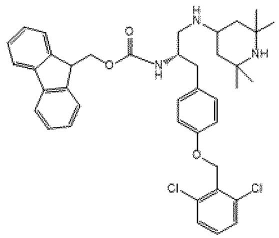 | 686.25 | $^1$H NMR (400 MHz, DMSO-$d_6$) $\delta$ 7.88 (t, *J* = 8.6 Hz, 3H), 7.64 (t, *J* = 7.7 Hz, 2H), 7.55 (d, *J* = 7.4 Hz, 2H), 7.52 - 7.27 (m, 5H), 7.19 (d, *J* = 8.7 Hz, 1H), 7.14 (d, *J* = 8.3 Hz, 2H), 6.93 (d, *J* = 8.0 Hz, 2H), 5.22 - 5.09 (m, 2H), 4.27 (dd, *J* = 8.6, 4.7 Hz, 1H), 4.22 - 4.12 (m, 2H), 3.65 (d, *J* = 6.5 Hz, 1H), 2.86 (s, 1H), 2.78 (dd, *J* = 13.6, 5.2 Hz, 1H), 2.59 (s, 12H), 1.98 (s, 1H), 1.76 (t, *J* = 12.3 Hz, 2H), 1.47 (s, 1H), 1.32 - 1.17 (m, 22H), 1.01 (s, 4H), 0.95 (dd, *J* = 13.8, 6.6 Hz, 1H) | (9*H*-fluoren-9-yl)methyl (*S*)-(1-(4-((2,6-dichloroben zyl) oxy)phenyl)-3-((2,2,6,6 -tetramethylpiperidin-4-yl) amino)propan-2-yl)carbam ate |
| Cpd 007 | 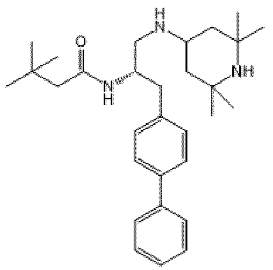 | 464.3 | $^1$H NMR (400 MHz, DMSO-$d_6$) $\delta$ 8.17 (s, 1H), 7.69 - 7.56 (m, 3H), 7.59 - 7.53 (m, 3H), 7.45 (dd, J = 8.3, 7.0 Hz, 2H), 7.34 (t, J = 7.4 Hz, 1H), 7.30 (d, J = 8.1 Hz, 2H), 4.05 (s, 1H), 3.30 (s, 2H), 2.95 (s, 2H), 2.88 (dd, J = 13.8, 5.5 Hz, 1H), 2.65 (dd, J = 13.8, 8.3 Hz, 1H), 2.60 (d, J = 6.2 Hz, 2H), 1.93 - 1.82 (m, 4H), 1.41 - 1.30 (m, 12H), 1.24 (s, 1H), 1.16 (s, 1H), 1.12 (d, J = 11.7 Hz, 1H), 0.87 (s, 9H). | (*S*)-*N*-(1-([1,1'-biphenyl]-4 -yl)-3-((2,2,6,6-tetramethyl piperidin-4-yl)amino)propa n-2-yl)-3,3-dimethylbutana mide |

(continued)

| No. | Structural formula | M+H | $^1$H NMR | Name |
|---|---|---|---|---|
| Cpd 008 | 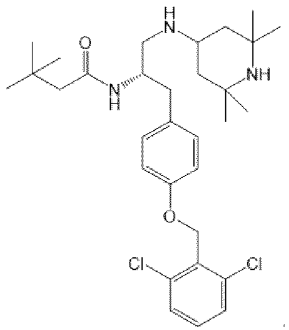 | 562.45 | $^1$H NMR (400 MHz, DMSO-$d_6$) δ 8.17 (s, 1H), 7.59 - 7.54 (m, 2H), 7.53 (s, 1H), 7.47 (dd, J = 8.9, 7.1 Hz, 1H), 7.15 (d, J = 8.6 Hz, 2H), 6.99 - 6.92 (m, 2H), 5.18 (s, 2H), 4.06 - 3.97 (m, 1H), 3.30 (s, 1H), 2.97 (s, 1H), 2.78 (dd, J = 13.9, 5.6 Hz, 1H), 2.61 - 2.52 (m, 3H), 1.96 - 1.82 (m, 4H), 1.37 - 1.31 (m, 11H), 1.28 - 1.17 (m, 1H), 1.20 - 1.12 (m, 1H), 0.87 (s, 8H). | (*S*)-*N*-(1-(4-((2,6-dichlorob enzyl) oxy)phenyl)-3-((2,2, 6,6-tetra-methylpiperidin-4-yl)amino)pro-pan-2-yl)-3,3-dimethylbutanamide |
| Cpd 009 | 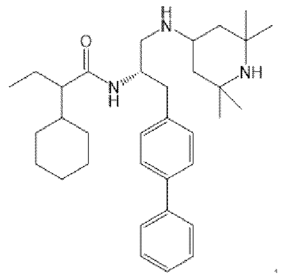 | 518.55 | $^1$H NMR (400 MHz, DMSO-$d_6$) δ 8.32 (s, 1H), 7.68 - 7.51 (m, 6H), 7.44 (t, J = 7.6 Hz, 2H), 7.32 (ddd, J = 18.0, 7.7, 1.9 Hz, 4H), 4.12 (s, 1H), 2.98 (d, J = 12.6 Hz, 1H), 2.90 (dt, J = 20.0, 7.0 Hz, 1H), 2.70 - 2.54 (m, 4H), 1.87 (s, 2H), 1.77 - 1.65 (m, 1H), 1.65 - 1.49 (m, 2H), 1.33 (t, J = 7.4 Hz, 17H), 1.27 - 1.21 (m, 2H), 1.10 (t, J = 14.2 Hz, 5H), 0.96 - 0.87 (m, 1H), 0.87 - 0.74 (m, 1H), 0.73 (d, J = 7.3 Hz, 1H), 0.62 (t, J = 12.6 Hz, 1H), 0.47 (t, J = 7.3 Hz, 2H). | *N*-((*S*)-1-([1,1'-biphenyl]-4 -yl)-3-((2,2,6,6-tetramethyl piperidin-4-yl)amino)propa n-2-yl)-2-cy-clohexylbutana mide |
| Cpd 010 | 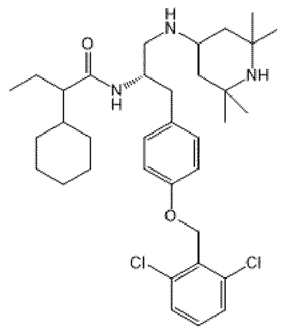 | 618.25 | $^1$H NMR (400 MHz, DMSO-$d_6$) δ 8.32 (s, 1H), 7.59 - 7.50 (m, 3H), 7.47 (dd, *J* = 9.0, 7.1 Hz, 1H), 7.15 (d, *J* = 8.5 Hz, 2H), 6.97 - 6.90 (m, 2H), 5.15 (s, 2H), 4.06 (s, 1H), 2.95 (s, 1H), 2.85 (dd, *J* = 13.8, 4.5 Hz, 1H), 2.58 (d, *J* = 6.3 Hz, 2H), 1.86 (t, *J* = 14.0 Hz, 2H), 1.73 - 1.55 (m, 3H), 1.49 (d, *J* = 9.2 Hz, 2H), 1.40 (d, *J* = 7.7 Hz, 1H), 1.36 - 1.21 (m, 14H), 1.14 (s, 7H), 1.04 (d, *J* = 12.0 Hz, 1H), 0.95 (dd, *J* = 10.9, 7.9 Hz, 1H), 0.74 (t, *J* = 7.3 Hz, 3H), 0.65 - 0.55 (m, 1H). | 2-cyclohexyl-*N*-((*S*)-1-(4-( (2,6-di-chlorobenzyl)oxy)p he-nyl)-3-((2,2,6,6-tetramet hylpiperidin-4-yl)amino)pr opan-2-yl)buta-namide |

(continued)

| No. | Structural formula | M+H | $^1$H NMR | Name |
|---|---|---|---|---|
| Cpd 011 | 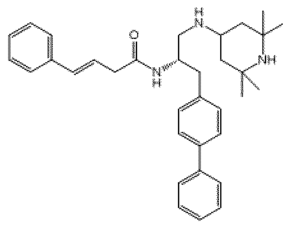 | 510.45 | $^1$H NMR (400 MHz, DMSO-$d_6$) δ 8.18 (s, 1H), 7.79 (d, *J* = 8.5 Hz, 1H), 7.60 - 7.49 (m, 4H), 7.43 (dd, *J* = 8.3, 6.9 Hz, 2H), 7.41 - 7.29 (m, 3H), 7.33 - 7.23 (m, 4H), 7.26 - 7.17 (m, 1H), 6.41 (d, *J* = 15.9 Hz, 1H), 6.24 (dt, *J* = 15.8, 7.0 Hz, 1H), 4.04 - 3.98 (m, 1H), 3.07 - 2.92 (m, 2H), 2.95 (s, 1H), 2.90 (dd, *J* = 13.7, 5.2 Hz, 1H), 2.72 - 2.58 (m, 3H), 1.87 (ddd, *J* = 21.3, 13.0, 3.3 Hz, 2H), 1.35 - 1.21 (m, 13H), 1.14 (q, *J* = 11.8 Hz, 2H). | (*S*,*E*)-*N*-(1-([1,1'-biphenyl] -4-yl)-3-((2,2,6,6-tetrameth ylpiperidin-4-yl)amino)pro pan-2-yl)-4-phenylbut-3-en amide |
| Cpd 012 | 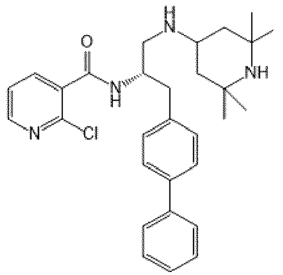 | 505.3 | $^1$H NMR (400 MHz, DMSO-$d_6$) δ 8.52 - 8.42 (m, 2H), 7.70 (dd, *J* = 7.6, 2.0 Hz, 1H), 7.66 (dd, *J* = 7.2, 1.8 Hz, 2H), 7.63 - 7.58 (m, 2H), 7.52 - 7.42 (m, 3H), 7.36 (d, *J* = 7.8 Hz, 3H), 4.26 - 4.16 (m, 1H), 2.98 (dd, *J* = 13.7, 5.5 Hz, 2H), 2.90 (s, 0H), 2.77 (dd, *J* = 13.7, 8.4 Hz, 1H), 2.73 (s, 1H), 2.72 (s, 1H), 1.85 (s, 2H), 1.30 - 1.21 (m, 14H), 1.06 (s, 3H). | (*S*)-*N*-(1-([1,1'-biphenyl]-4 -yl)-3-((2,2,6,6-tetramethyl piperidin-4-yl)amino)propa n-2-yl)-2-chloronicotinami de |
| Cpd 013 | 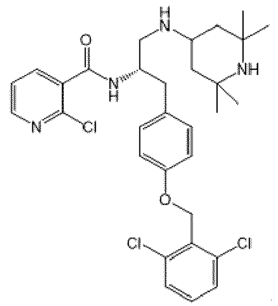 | 605.35 | $^1$H NMR (400 MHz, DMSO-$d_6$) δ 8.49 - 8.40 (m, 2H), 8.32 (s, 1H), 7.69 (dd, *J* = 7.5, 2.0 Hz, 1H), 7.57 (d, *J* = 8.0 Hz, 2H), 7.48 (ddd, *J* = 9.0, 6.0, 4.0 Hz, 2H), 7.21 (d, *J* = 8.2 Hz, 2H), 7.00 (d, *J* = 8.5 Hz, 2H), 5.20 (s, 2H), 4.15 (d, *J* = 7.7 Hz, 1H), 3.30 (s, 1H), 3.01 (s, 1H), 2.93 - 2.83 (m, 1H), 2.69 (dt, *J* = 13.7, 9.1 Hz, 3H), 2.03 - 1.85 (m, 2H), 1.35 (dd, *J* = 7.9, 2.7 Hz, 11H), 1.28 - 1.17 (m, 2H), 1.14 (d, *J* = 13.1 Hz, 1H). | (S)-2-chloro-N-(1-(4-((2,6-dichlorobenzyl)oxy)phenyl )-3-((2,2,6,6-tetramethylpi peridin-4-yl)amino) propan -2-yl)nicotinamide |
| Cpd 014 | 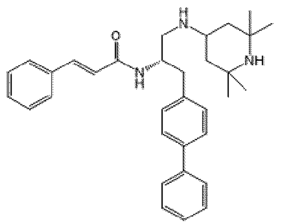 | 496.35 | $^1$H NMR (400 MHz, DMSO-$d_6$) δ 8.03 (d, *J* = 8.3 Hz, 1H), 7.79 (s, 4H), 7.69 - 7.61 (m, 2H), 7.56 (dd, *J* = 18.4, 7.5 Hz, 4H), 7.51 - 7.29 (m, 9H), 6.63 (d, *J* = 15.8 Hz, 1H), 4.14 (dt, *J* = 8.1, 5.3 Hz, 1H), 2.96 (dd, *J* = 13.9, 5.8 Hz, 2H), 2.76 (dd, *J* = 13.8, 8.0 Hz, 1H), 2.68 (d, *J* = 6.2 Hz, 2H), 2.58 (s, 0H), 1.88 (t, *J* = 11.2 Hz, 2H), 1.33 - 1.21 (m, 13H), 1.12 *(d, J*= 11.3 Hz, 2H). | (*S*)-*N*-(1-([1,1'-biphenyl]-4 -yl)-3-((2,2,6,6-tetramethyl piperidin-4-yl)amino)propa n-2-yl)cinna-mamide |

(continued)

| No. | Structural formula | M+H | $^1$H NMR | Name |
|---|---|---|---|---|
| Cpd 015 | 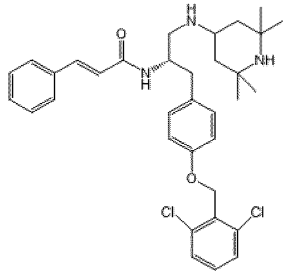 | 594.25 | $^1$H NMR (400 MHz, DMSO-$d_6$) δ 7.97 (d, $J$ = 8.4 Hz, 1H), 7.62 - 7.51 (m, 4H), 7.50 - 7.34 (m, 5H), 7.22 - 7.15 (m, 2H), 7.01 - 6.94 (m, 2H), 6.62 (d, $J$ = 15.8 Hz, 1H), 5.18 (s, 2H), 4.08 (q, $J$ = 6.7 Hz, 1H), 2.97 (s, 1H), 2.85 (dd, $J$ = 13.8, 5.7 Hz, 1H), 2.67 (dd, $J$ = 13.5, 7.4 Hz, 3H), 1.97 - 1.84 (m, 2H), 1.33 (d, $J$ = 7.9 Hz, 11H), 1.24 (d, $J$ = 3.4 Hz, 1H), 1.16 (q, $J$ = 11.7 Hz, 2H). | (*S*)-*N*-(1-(4-((2,6-dichlorob enzyl) oxy)phenyl)-3-((2,2, 6,6-tetra-methylpiperidin-4-yl)amino)pro-pan-2-yl)cinn amamide |
| Cpd 016 | 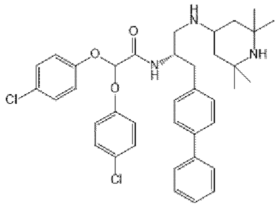 | 660.15 | $^1$H NMR (400 MHz, DMSO-$d_6$) δ 8.41 (d, $J$ = 8.7 Hz, 1H), 7.64 (d, $J$ = 7.6 Hz, 2H), 7.56 (d, $J$ = 7.9 Hz, 2H), 7.46 (t, $J$ = 7.6 Hz, 2H), 7.39 - 7.25 (m, 8H), 7.04 - 6.92 (m, 5H), 6.17 (s, 1H), 4.09 (d, $J$ = 8.3 Hz, 1H), 2.98 - 2.89 (m, 2H), 2.77 (dd, $J$ = 13.8, 8.9 Hz, 1H), 2.68 (d, $J$ = 6.4 Hz, 2H), 1.87 (d, $J$ = 13.3 Hz, | (*S*)-*N*-(1-([1,1'-biphenyl]-4 -yl)-3-((2,2,6,6-tetramethyl piperidin-4-yl)amino)propa n-2-yl)-2,2-bis(4-chlorophe noxy)acetamide |
| | | | 1H), 1.77 (d, $J$ = 13.8 Hz, 1H), 1.61 (s, 1H), 1.32 (d, $J$ = 6.6 Hz, 6H), 1.30 (s, 6H), 1.25 (d, $J$ = 8.7 Hz, 1H), 1.13 (q, $J$ = 11.3, 10.8 Hz, 2H). | |
| Cpd 017 | 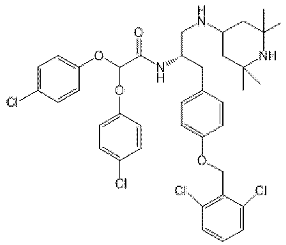 | 760.35 | $^1$H NMR (400 MHz, DMSO-$d_6$) δ 8.33 (d, $J$ = 8.7 Hz, 1H), 7.56 (d, $J$ = 8.0 Hz, 2H), 7.51 - 7.43 (m, 1H), 7.35 (t, $J$ = 9.1 Hz, 4H), 7.16 (d, $J$ = 8.2 Hz, 2H), 7.05 - 6.93 (m, 7H), 6.16 (s, 1H), 5.20 (s, 2H), 4.01 (q, $J$ = 7.3 Hz, 1H), 2.91 (s, 2H), 2.83 (dd, $J$ = 13.7, 5.1 Hz, 1H), 2.67 (dd, $J$ = 23.6, 7.6 Hz, 3H), 1.85 (d, $J$ = 13.4 Hz, 1H), 1.76 (d, $J$ = 13.0 Hz, 1H), 1.35 - 1.27 (m, 12H), 1.24 (s, 1H), 1.21 - 1.03 (m, 2H). | (*S*)-2,2-bis(4-chlorophenox y)-*N*-(1-(4-((2,6-dichlorobe nzyl) oxy)phenyl)-3-((2,2,6, 6-tetra-methylpiperidin-4-yl )amino)pro-pan-2-yl)aceta mide |

As used herein, the term "alkyl" refers to a group consisting solely of carbon and hydrogen atoms, and having no unsaturation (e.g. double bonds, triple bonds, or rings), and encompasses the various possible geometric and stereoisomeric groups. The group is attached to the rest of the molecule by a single bond. As non-limiting examples of alkyl groups, one may cite the following linear or branched groups: methyl, ethyl, n-propyl, isopropyl, n-butyl, isobutyl, sec-butyl, tert-butyl, n-pentyl and its seven other isomers, n-hexyl and its sixteen other isomers, n-heptyl and its various isomers, n-octyl and its various isomers, n-nonyl and its various isomers, n-decyl and its various isomers.

As used herein, the term "cycloalkyl" refers to a saturated non-aromatic ring system composed of at least 3 carbon atoms, which may be monocyclic, bicyclic, polycyclic, or fused, bridged, spiro. As non-limiting examples of cycloalkyl, one may cite the following groups: cyclopropyl, cyclobutyl, cyclopentyl, cyclohexyl, cycloheptyl, cyclooctyl, cyclononyl, cyclodecyl; and fused, bridged, or spiro groups formed from two or more of the above monocyclic rings through a common edge and a common carbon atom.

As used herein, the term "aryl", employed alone or as part of an "arylalkyl" group, refers to monocyclic, bicyclic, and tricyclic carbocyclic ring systems containing a total of six to fourteen members, wherein at least one of the ring systems is aromatic, wherein each ring system contains three to seven ring members and only one point of attachment is to the rest of the molecule. The term "aryl" may be used interchangeably with the term "aromatic ring", e.g. the aromatic ring may include phenyl, naphthyl, anthracenyl.

As used herein, the term "heteroaryl" refers to a 5-14 membered aromatic heterocyclic ring system having one or more heteroatoms independently selected from N, O, or S, which ring system may be monocyclic, bicyclic, polycyclic, wherein bicyclic and polycyclic rings may be formed from monocyclic rings joined by single bonds or fused. As non-limiting examples of heteroaryl groups one may cite the following groups: oxazolyl, isoxazolyl, imidazolyl, furanyl, indolyl, isoindolyl, pyrrolyl, triazolyl, triazinyl, tetrazolyl, thienyl, thiazolyl, isothiazolyl, pyridinyl, pyrimidinyl, pyrazinyl, pyridazinyl, benzofuranyl, benzothiazolyl, benzoxazolyl, benzimidazolyl, benzothienyl, benzopyranyl, carbazolyl, quinolinyl, isoquinolinyl, quinazolinyl, cinnolinyl, naphthyridinyl, pteridinyl, purinyl, quinoxalinyl, thiadiazolyl, indolizinyl, acridinyl, phenazinyl, phthalazinyl, coumarinyl, pyrazolopyridinyl, pyridopyridazinyl, pyrrolopyridinyl, imidazopyridinyl, pyrazolopyridazinyl; and a group formed from the above-mentioned heteroaryl group by a single bond connection means or a fusion means.

The compounds of the present application may also be used in the form of their pharmaceutically acceptable salts, esters, solvates, or isomers, including stereoisomers, enantiomers, tautomers, or mixtures thereof.

Another object of the present application is to provide a preparation method for ethylenediamine derivatives represented by Formula I.

The synthetic route of ethylenediamine derivatives of Formula I provided by the present application is as follows:

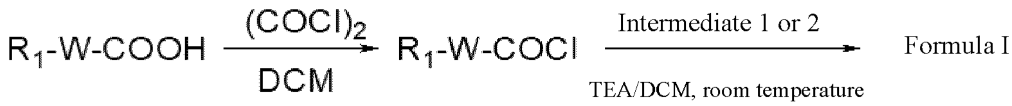


A specific preparation method, including the steps of: activation of $R_1$-W-COOH with oxalyl chloride [$(COCl)_2$)] followed by reaction with Intermediate 1 or Intermediate 2 at room temperature to afford the corresponding final products of Formula I.

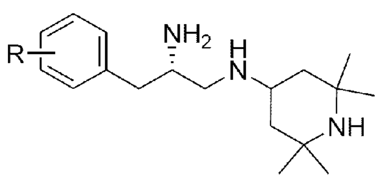

Intermediate 1

Wherein R in Intermediate 1 is -X-$R_2$, wherein X is defined as in Formula I (excluding the case where X is -$OCH_2$-), and wherein $R_2$ in Intermediate 1 is defined as in Formula I.

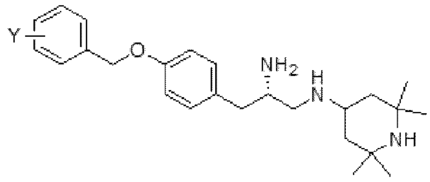

Intermediate 2

In the Intermediate 2, Y represents a group in which the hydrogen in the phenyl ring to which Y is attached is substituted by one or more $R^{15}$, wherein $R^{15}$ is defined as for $R^1$ in Formula I.

In particular, the synthetic route of the Intermediate 1 is shown below:

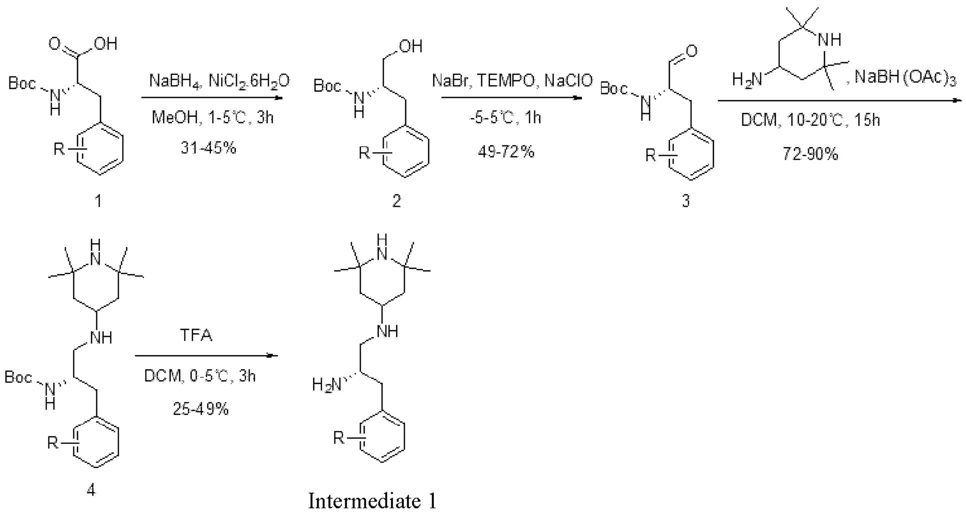


Intermediate 1

A specific preparation method for the Intermediate 1 is as follows: reducing carboxylic acid 1 under Lewis acid catalysis and $NaBH_4$ to obtain compound 2; oxidizing compound 2 in the conditions of NaBr, NaClO, and TEMPO to obtain compound 3; followed by reductive amination with 4-amino-2, 2, 6, 6-tetramethylpiperidine under the conditions of $NaBH(OAc)_3$ to obtain compound 4; and deprotecting compound 4 by TFA in DCM to obtain Intermediate 1.

The synthetic route of the Intermediate 2 is shown below:

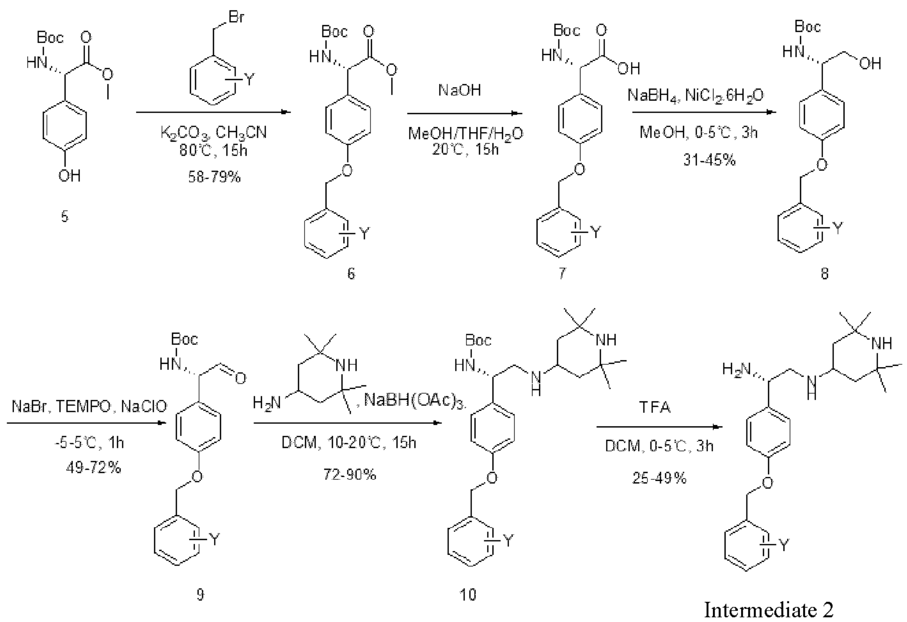


Intermediate 2

A specific preparation method for the Intermediate 2 is as follows: performing a substitution reaction of phenol 5

and the bromide under the condition of $K_2CO_3$ to obtain a compound 6; hydrolyzing compound 6 by NaOH in MeOH/THF/$H_2O$ solution to obtain compound 7; reducing compound 7 under the conditions of $NaBH_4$ to obtain compound 8; oxidizing compound 8 by NaBr, TEMPO, and NaClO again to obtain compound 9; subjecting the compound 9 to a reductive amination reaction with 4-amino-2, 2, 6, 6-tetramethylpiperidine to obtain a compound 10; and deprotecting compound 10 via TFA to obtain Intermediate 2.

It is a further object of the present application to provide the use of an ethylenediamine derivative of Formula I as described above, or a pharmaceutically acceptable salt, ester, solvate, or isomer thereof, including stereoisomers, enantiomers, tautomers, or mixtures thereof.

Uses provided by the present application include the following: 1) Use of an ethylenediamine derivative represented by Formula I, or a pharmaceutically acceptable salt, ester, solvate, or isomer thereof (including a stereoisomer, enantiomer, tautomer, or mixture thereof), for the manufacture of a drug for the prevention and/or treatment of cancer; 2) Use of an ethylenediamine derivative represented by Formula I, or a pharmaceutically acceptable salt, ester, solvate, or isomer thereof, including a stereoisomer, enantiomer, tautomer, or mixture thereof, for the manufacture of a drug for inhibiting proliferation of cancer cells.

The cancers include various cancers (solid or non-solid) known in the art, including, but not limited to: liver cancer, lung cancer, and prostate cancer.

The cancer cells include liver cancer cells (e.g. Bel-7402 cells, HepG-2 cells, SK-hep1 cells), lung cancer cells (e.g. A549 cells, H460 cells, H1299 cells, H292 cells), prostate cancer cells (e.g. PC-3 cells).

A drug for the prevention and/or treatment of cancer, which is prepared using an ethylenediamine derivative represented by Formula I or a pharmaceutically acceptable salt, ester, solvate, or isomer thereof (including stereoisomer, enantiomer, tautomer, or mixture thereof) as an active ingredient falls within the scope of the present application.

**Detailed Description of the Invention**

Although the present application has been described in detail with reference to the preferred embodiments, it is to be understood that the present application is not limited to the disclosed embodiments, but, on the contrary, is intended to cover various modifications and equivalent arrangements included within the spirit and scope of the appended claims.

The experimental methods used in the following examples are conventional unless otherwise specified.

The materials, reagents, and the like used in the following examples are commercially available unless otherwise specified.

The structural formula of the PL-AC-15 compound referred to in the following examples is shown below:

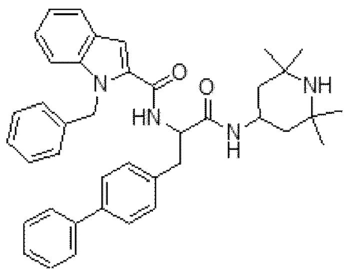

Chinese Patent No. CN107382827B is referred for the details of its preparation.

The structural formula of the PL-AC-202 compound referred to in the following examples is shown below:

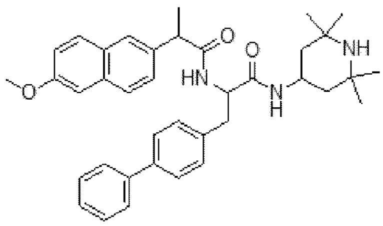

Chinese Patent No. CN113620862A is referred for the details of its preparation.

I. Preparation and characterization of the compounds

Example 1:

(S, E)-N-(1-(4-((2,6-dichlorobenzyl) oxy) phenyl)-3-((2,2,6,6-tetramethylpiperidin-4-yl) amino) propan -2-yl)-4-phenyl-3-enamide (Cpd001)

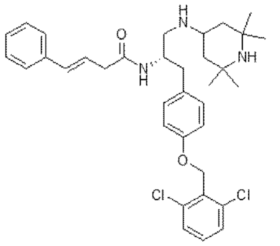

The preparation method for Intermediate 16 (corresponding to Y = 2,6-dichloro in Intermediate 2) is as follows:

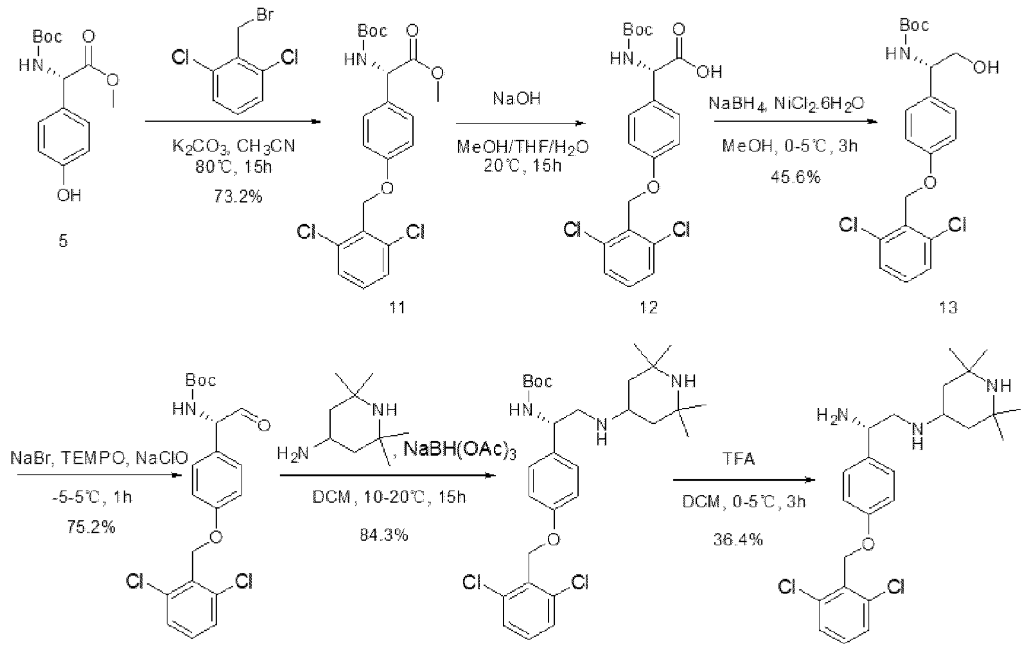

5 11 12 13
14 15 Intermediate 16

1) Compound 5 (47.8 g, 170 mmol) was dissolved in acetonitrile, potassium carbonate (48.4 g, 350 mmol) was added, the mixture was stirred for 10 min, and then 2,6-dichlorobenzyl bromide (33.6 g, 140 mmol) was added, and the mixture was reacted at 80°C for 15 h. After completion of compound 5 as shown by TLC (EA/PE = 1/5, v/v), the system was spin-dried, 300 mL of water was added to obtain white insoluble material and suction filtration was performed to obtain compound 11 (45 g) as a white solid.

2) Compound 11 (35.0 g, 79 mmol) was dissolved in tetrahydrofuran and methanol (350 mL: 350 mL, v/v), aqueous sodium hydroxide solution was slowly added, and the reaction was performed at room temperature for 15 h. After completion of compound 11 as shown by TLC (EA/PE = 1/5, v/v), the reaction mixture was spin-dried, water was added, the pH of the reaction mixture was adjusted to 3-4 with dilute hydrochloric acid, and suction filtration was performed to obtain compound 12 as a white solid (23 g, yield 68.4%).

3) Compound 12 (21.3 g, 50 mmol) was dissolved in methanol, cooled to 0-5°C, $NiCl_2 \cdot 6H_2O$ (11.9 g, 50 mmol) was added with stirring, followed by $NaBH_4$ (7.6 g, 200 mmol) in portions. After the addition was completed, the mixture was kept warm and reacted for more than 1 h, the reaction of compound 12 was shown to be complete by TLC (EA/PE = 1/3, v/v). The system was filtered directly by suction to obtain a dark gray filtrate. Saturated sodium bicarbonate solution was added to the filtrate to adjust the pH of the system to 7-8. It was extracted with ethyl acetate and spin-dried to obtain a pale-yellow oil, which was purified by column chromatography (EA/PE = 1/5, v/v) to obtain compound 13 (9.4 g, yield 45.6%).

4) Compound 13 (8.5 g, 20 mmol) was dissolved in isopropyl acetate, sodium bromide (206 mg, 2 mmol) and TEMPO (31.3 mg, 0.2 mmol) were added, the mixture was cooled to -5-5°C, and an aqueous solution of sodium hypochlorite

(2.2 g, 30 mmol) was adjusted to pH 9-10 with sodium bicarbonate was added dropwise. About 1 h after the end of the drop, the reaction was shown to be complete by TLC (EA/PE = 1/3, v/v). The reaction was brought back to room temperature, quenched by the addition of 10% sodium sulfite solution, and extracted with ethyl acetate, the organic phase was spin-dried to dryness to obtain a pale-yellow oil 14 (6.2 g, 75.2% yield).

5) Compound 14 (14.0g, 34 mmol) was dissolved in dichloromethane, 4-amino-2,2,6,6-tetramethylpiperidine (5.3 g, 34 mmol) was added, cooled to around 0°C, $NaBH(OAc)_3$ (14.4 g, 68 mmol) was added in portions after the addition was completed, the reaction was allowed to return to room temperature for 15 h. After completion of the reaction as shown by TLC (EA/PE = 1/3, v/v), saturated sodium bicarbonate solution was added to adjust the pH of the system to 7-8, which was extracted with dichloromethane, and the organic phase was spin-dried to obtain 15 (15.8 g, 84.3% yield) as pale-yellow oil.

6) Compound 15 (15.0 g, 27 mmol) was dissolved in 500 mL of dichloromethane, cooled to 0-5°C, and 100 mL TFA was added dropwise. After the addition was completed, the mixture was kept warm and under stirring for 30 min, and the reaction was allowed to return to room temperature for 3 h. After completion of the reaction as shown by TLC (methanol/dichloromethane = 1/10, v/v), the system was spin-dried directly, saturated sodium bicarbonate solution was added to adjust the pH of the system to 7-8, and extracted with ethyl acetate. After drying the spin-dried organic phase, it was purified by column chromatography (methanol/dichloromethane = 1/20, v/v) to obtain Intermediate 16 (4.3 g, yield 36.4%) as a light reddish brown clear oil.

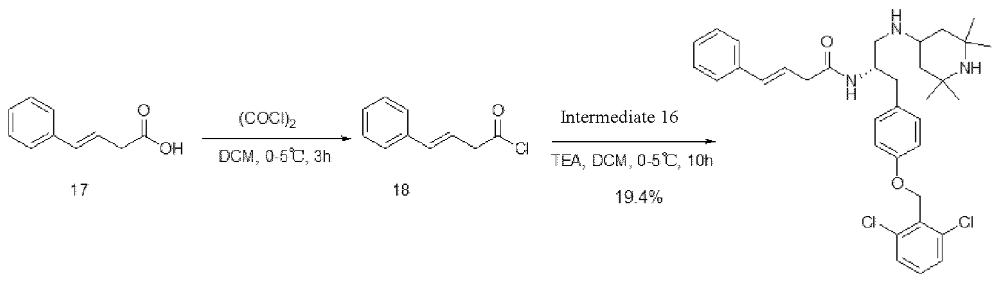

Example 1 (Cpd001)

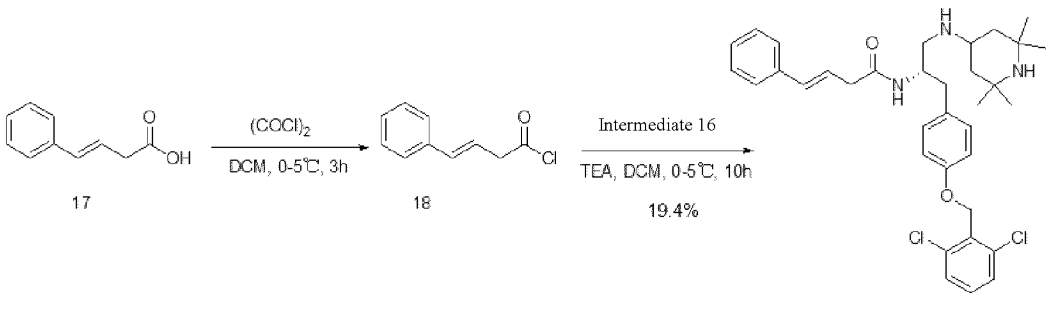

Example 1 (Cpd001)

1) Compound 17 (0.73 g, 4.5 mmol) was dissolved in 10 mL of dichloromethane, 3 drops of DMF were added dropwise, cooled to 0-5°C, oxalyl chloride (1.14 g, 9 mmol) was added dropwise, after the end of the dropwise addition, the mixture was kept warm and under stirring for 20 min, and then returned to room temperature for 5 h. After the reaction, the system was spin-dried for ready use to obtain Intermediate 18.

2) Intermediate 18 (0.81g, 4.5 mmol) was dissolved in 10 mL of dichloromethane, triethylamine was added, cooled to 0-5°C, 10 mL of a solution of the product of step (Intermediate 16, 2.03 g, 4.5 mmol) in dichloromethane was slowly added dropwise, after the dropwise addition was completed, the mixture was kept warm for 30 min, and the reaction was allowed to return to room temperature for 10 h. The reaction was shown to be complete by TLC (methanol/dichloromethane = 1/10, v/v). The reaction solution was washed with water, the organic phase was spin-dried to dryness, and purified by column chromatography (methanol/dichloromethane = 1/15, v/v) to obtain the product Cpd001 (S, E)-N-(1-(4-((2,6-dichlorobenzyl) oxy) phenyl)-3-((2,2,6,6-tetramethylpiperidin-4-yl) amino) propan -2-yl)-4-phenyl-3-enamide (530 mg, yield 19.4%).

Structural elucidation data: $^1H$ NMR (400 MHz, DMSO-$d_6$) δ 8.12 (s, 2H), 7.74 (d, $J$ = 8.2 Hz, 1H), 7.59 - 7.53 (m, 2H), 7.47 (dd, $J$ = 8.9, 7.1 Hz, 1H), 7.39 (d, $J$ = 7.2 Hz, 2H), 7.36 - 7.28 (m, 2H), 7.26 - 7.16 (m, 1H), 7.19 - 7.12 (m, 2H), 6.96 - 6.88 (m, 2H), 6.44 (d, $J$ = 15.9 Hz, 1H), 6.28 (dt, $J$ = 15.8, 7.0 Hz, 1H), 5.20 - 5.08 (m, 2H), 3.95 (s, 1H), 3.30 (s, 1H), 3.11 (s,

1H), 3.00 (d, *J* = 7.1 Hz, 2H), 2.94 (s, 1H), 2.78 (dd, *J* = 13.8, 5.3 Hz, 1H), 2.61 (t, *J* = 7.0 Hz, 3H), 1.85 (t, *J* = 16.1 Hz, 2H), 1.34 - 1.21 (m, 13H), 1.13 (d, *J* = 12.2 Hz, 2H). MS m/z: 608.2 (M+H).

Other compounds of Formula I were prepared following the preparation method described above for example 1 (Cpd001) (racemic) using the corresponding intermediates and carboxylic acids.

Example 2:

(*S*)-*N*-((*S*)-1-([1,1'-biphenyl]-4-yl)-3-((2,2,6,6-tetramethylpiperidin-4-yl) amino)propan -2-yl)-2-(2-fluoro-[1,1'-biphenyl]-4-yl) propanamide (Cpd002)

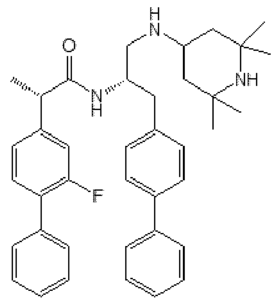

$^1$H NMR (400 MHz, $CDCl_3$) δ 7.58 - 7.29 (m, 13H), 7.20 (d, *J* = 7.9 Hz, 2H), 7.16 - 7.08 (m, 2H), 6.26 (s, 1H), 4.24 (d, *J* = 7.0 Hz, 1H), 3.61 (q, *J* = 7.1 Hz, 1H), 3.48 (s, 2H), 2.92 (dd, *J* = 13.7, 6.7 Hz, 1H), 2.81 (dd, *J* = 13.8, 7.1 Hz, 1H), 2.69 (s, 1H), 1.78 (d, *J* = 13.1 Hz, 1H), 1.65 (d, *J* = 13.1 Hz, 1H), 1.52 - 1.44 (m, 7H), 1.40 (d, *J* = 8.3 Hz, 6H), 1.33 (s, 3H), 1.25 (t, *J* = 3.5 Hz, 1H), 0.86 (s, 1H), 0.07 (s, 1H). MS m/z: 592.4 (M+H).

Example 3:

(S)-2-(2-Fluoro-[1,1'-biphenyl]-4-yl)-N-((S)-1-phenyl-3-((2,2,6,6-tetramethylpiperidin-4-yl) amino) propan -2-yl) propanamide (Cpd003)

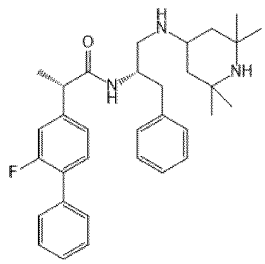

$^1$H NMR (400 MHz, DMSO-$d_6$) δ 7.83 (d, *J* = 8.4 Hz, 1H), 7.57 - 7.45 (m, 4H), 7.47 - 7.34 (m, 2H), 7.15 - 7.04 (m, 5H), 7.02 (dd, *J* = 7.3, 2.2 Hz, 2H), 3.93 (d, *J* = 6.3 Hz, 1H), 3.62 (q, *J* = 7.0 Hz, 1H), 2.86 - 2.74 (m, 2H), 2.57 (dd, *J* = 13.4, 7.6 Hz, 3H), 1.70 (t, *J* = 13.9 Hz, 2H), 1.32 (d, *J* = 7.0 Hz, 3H), 1.28 - 1.21 (m, 2H), 1.15 (s, 6H), 1.08 (s, 6H), 1.04 (s, 0H), 0.89 - 0.82 (m, 3H).MS m/z: 592.4 (M+H).

Example 4:

(S)-N-((S)-1-(4-((2,6-dichlorobenzyl)oxy) phenyl)-3-((2,2,6,6-tetramethylpiperidin-4-yl) amino) propan -2-yl)-2-(2-fluoro-[1,1'-biphenyl]-4-yl) propanamide (Cpd004)

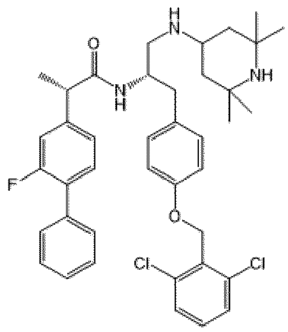

$^1$H NMR (400 MHz, DMSO-$d_6$) δ 7.85 (d, *J* = 8.7 Hz, 1H), 7.60 - 7.49 (m, 4H), 7.53 - 7.42 (m, 4H), 7.46 - 7.35 (m, 1H), 7.27 - 7.19 (m, 2H), 7.18 - 7.11 (m, 2H), 7.01 - 6.92 (m, 2H), 5.18 (s, 2H), 3.95 (d, *J* = 7.1 Hz, 1H), 3.63 (q, *J* = 7.0 Hz, 1H), 3.29 (s, 1H), 2.83 (s, 1H), 2.79 (dd, *J* = 13.7, 5.3 Hz, 1H), 2.66 - 2.56 (m, 2H), 2.53 (s, 1H), 1.82 - 1.73 (m, 1H), 1.58 (d, *J* = 13.1 Hz, 1H), 1.30 - 1.24 (m, 6H), 1.23 (d, *J* = 16.0 Hz, 10H), 1.03 (s, 2H). MS m/z: 690.3 (M+H).

Example 5:

(S)-N-((S)-1-(4-chlorophenyl)-3-((2,2,6,6-tetramethylpiperidin-4-yl) amino) propan -2-yl)-2-(2-fluoro-[1,1'-biphenyl]-4-yl) propanamide (Cpd005)

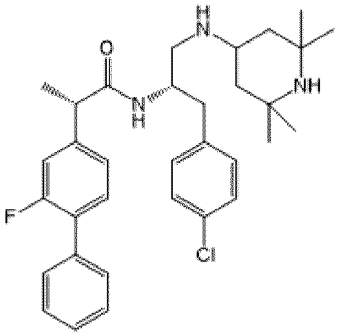

$^1$H NMR (400 MHz, DMSO-$d_6$) δ 7.85 (dd, J = 12.4, 8.6 Hz, 1H), 7.58 - 7.29 (m, 10H), 7.31 (s, 1H), 7.25 - 7.17 (m, 3H), 7.14 - 6.99 (m, 3H), 3.95 (s, 2H), 3.60 (dd, J = 7.2, 3.9 Hz, 1H), 2.82 (dt, J = 11.8, 5.7 Hz, 2H), 2.70 (s, 1H), 2.67 - 2.53 (m, 3H), 2.05 - 1.95 (m, 1H), 1.72 (t, J = 15.9 Hz, 1H), 1.61 (d, J = 12.4 Hz, 1H), 1.50 - 1.41 (m, 1H), 1.31 (d, J = 7.5 Hz, 3H), 1.29 - 1.21 (m, 8H), 1.17 (s, 4H), 1.10 (d, J = 5.8 Hz, 6H), 1.05 (s, 4H), 1.00 (s, 3H), 0.85 (t, J = 6.6 Hz, 1H), 0.75 (s, 2H). MS m/z: 550.25 (M+H).

Example 6:

(9H-fluoren-9-yl)methyl-(1-(4-((2,6-dichlorobenzyl)oxy) phenyl)-3-((2,2,6,6-tetramethylpiperidin-4-yl) amino) propan -2-yl) carbamate (Cpd006)

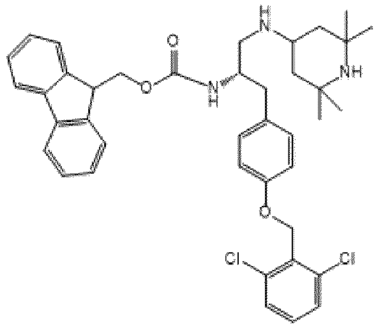

$^{1}H$ NMR (400 MHz, DMSO-$d_6$) δ 7.88 (t, $J$ = 8.6 Hz, 3H), 7.64 (t, $J$ = 7.7 Hz, 2H), 7.55 (d, $J$ = 7.4 Hz, 2H), 7.52 - 7.27 (m, 5H), 7.19 (d, $J$ = 8.7 Hz, 1H), 7.14 (d, $J$ = 8.3 Hz, 2H), 6.93 (d, $J$ = 8.0 Hz, 2H), 5.22 - 5.09 (m, 2H), 4.27 (dd, $J$ = 8.6, 4.7 Hz, 1H), 4.22 - 4.12 (m, 2H), 3.65 (d, $J$ = 6.5 Hz, 1H), 2.86 (s, 1H), 2.78 (dd, $J$ = 13.6, 5.2 Hz, 1H), 2.59 (s, 12H), 1.98 (s, 1H), 1.76 (t, $J$ = 12.3 Hz, 2H), 1.47 (s, 1H), 1.32 - 1.17 (m, 22H), 1.01 (s, 4H), 0.95 (dd, $J$ = 13.8, 6.6 Hz, 1H). MS m/z: 686.25 (M+H).

Example 7:

(S)-N-(1-([1,1'-biphenyl]-4-yl)-3-((2,2,6,6-tetramethylpiperidin-4-yl) amino) propan -2-yl)-3,3-dimethylbutylamine (Cpd007)

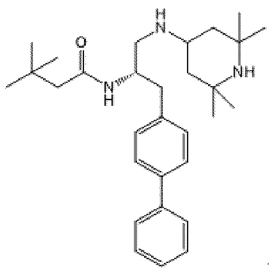

$^{1}H$ NMR (400 MHz, DMSO-$d_6$) δ 8.17 (s, 1H), 7.69 - 7.56 (m, 3H), 7.59 - 7.53 (m, 3H), 7.45 (dd, $J$ = 8.3, 7.0 Hz, 2H), 7.34 (t, $J$ = 7.4 Hz, 1H), 7.30 (d, $J$ = 8.1 Hz, 2H), 4.05 (s, 1H), 3.30 (s, 2H), 2.95 (s, 2H), 2.88 (dd, $J$ = 13.8, 5.5 Hz, 1H), 2.65 (dd, $J$ = 13.8, 8.3 Hz, 1H), 2.60 (d, $J$ = 6.2 Hz, 2H), 1.93 - 1.82 (m, 4H), 1.41 - 1.30 (m, 12H), 1.24 (s, 1H), 1.16 (s, 1H), 1.12 (d, $J$ = 11.7 Hz, 1H), 0.87 (s, 9H). MS m/z: 464.3 (M+H).

Example 8:

(S)-N-(1-(4-((2,6-dichlorobenzyl) oxy) phenyl)-3-((2,2,6,6-tetramethylpiperidin-4-yl) amino) propan -2-yl)-3,3-dimethylbutylamine (Cpd008)

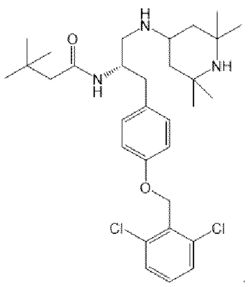

$^{1}H$ NMR (400 MHz, DMSO-$d_6$) δ 8.17 (s, 1H), 7.59 - 7.54 (m, 2H), 7.53 (s, 1H), 7.47 (dd, $J$ = 8.9, 7.1 Hz, 1H), 7.15 (d, $J$ = 8.6 Hz, 2H), 6.99 - 6.92 (m, 2H), 5.18 (s, 2H), 4.06 - 3.97 (m, 1H), 3.30 (s, 1H), 2.97 (s, 1H), 2.78 (dd, $J$ = 13.9, 5.6 Hz, 1H), 2.61 - 2.52 (m, 3H), 1.96 - 1.82 (m, 4H), 1.37 - 1.31 (m, 11H), 1.28 - 1.17 (m, 1H), 1.20 - 1.12 (m, 1H), 0.87 (s, 8H). MS

m/z: 562.45 (M+H).

Example 9:

N-((S)-1-([1,1'-biphenyl]-4-yl)-3-((2,2,6,6-tetramethylpiperidin-4-yl) amino) propan -2-yl)-2-cyclohexylbutylamine (Cpd009)

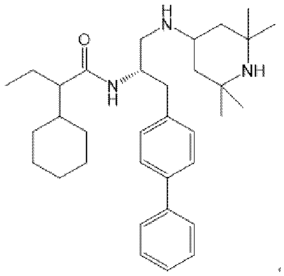

$^1$H NMR (400 MHz, DMSO-$d_6$) δ 8.32 (s, 1H), 7.68 - 7.51 (m, 6H), 7.44 (t, *J* = 7.6 Hz, 2H), 7.32 (ddd, *J* = 18.0, 7.7, 1.9 Hz, 4H), 4.12 (s, 1H), 2.98 (d, *J* = 12.6 Hz, 1H), 2.90 (dt, *J* = 20.0, 7.0 Hz, 1H), 2.70 - 2.54 (m, 4H), 1.87 (s, 2H), 1.77 - 1.65 (m, 1H), 1.65 - 1.49 (m, 2H), 1.33 (t, *J* = 7.4 Hz, 17H), 1.27 - 1.21 (m, 2H), 1.10 (t, *J* = 14.2 Hz, 5H), 0.96 - 0.87 (m, 1H), 0.87 - 0.74 (m, 1H), 0.73 (d, *J* = 7.3 Hz, 1H), 0.62 (t, *J* = 12.6 Hz, 1H), 0.47 (t, *J* = 7.3 Hz, 2H). MS m/z: 518.55 (M+H).

Example 10:

2-cyclohexyl-N-((S)-1-(4-((2,6-dichlorobenzyl) oxy) phenyl)-3-((2,2,6,6-tetramethylpiperidin-4-yl) amino) propan -2-yl) butanamide (Cpd010)

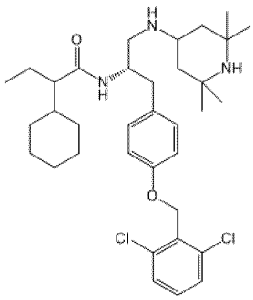

$^1$H NMR (400 MHz, DMSO-$d_6$) δ 8.32 (s, 1H), 7.59 - 7.50 (m, 3H), 7.47 (dd, *J* = 9.0, 7.1 Hz, 1H), 7.15 (d, *J* = 8.5 Hz, 2H), 6.97 - 6.90 (m, 2H), 5.15 (s, 2H), 4.06 (s, 1H), 2.95 (s, 1H), 2.85 (dd, *J* = 13.8, 4.5 Hz, 1H), 2.58 (d, *J* = 6.3 Hz, 2H), 1.86 (t, *J* = 14.0 Hz, 2H), 1.73 - 1.55 (m, 3H), 1.49 (d, *J* = 9.2 Hz, 2H), 1.40 (d, *J* = 7.7 Hz, 1H), 1.36 - 1.21 (m, 14H), 1.14 (s, 7H), 1.04 (d,*J* = 12.0 Hz, 1H), 0.95 (dd, *J* = 10.9, 7.9 Hz, 1H), 0.74 (t, *J* = 7.3 Hz, 3H), 0.65 - 0.55 (m, 1H). MS m/z: 618.25 (M+H).

Example 11: (S, E)-N-(1-([1,1'-biphenyl]-4-yl)-3-((2,2,6,6-tetramethylpiperidin-4-yl) amino) propan -2-yl)-4-phenyl-3-enamide (Cpd011)

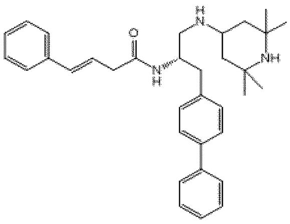

$^1$H NMR (400 MHz, DMSO-$d_6$) δ 8.18 (s, 1H), 7.79 (d, *J* = 8.5 Hz, 1H), 7.60 - 7.49 (m, 4H), 7.43 (dd, *J* = 8.3, 6.9 Hz, 2H), 7.41 - 7.29 (m, 3H), 7.33 - 7.23 (m, 4H), 7.26 - 7.17 (m, 1H), 6.41 (d, *J* = 15.9 Hz, 1H), 6.24 (dt, *J* = 15.8, 7.0 Hz, 1H), 4.04 - 3.98 (m, 1H), 3.07 - 2.92 (m, 2H), 2.95 (s, 1H), 2.90 (dd, *J* = 13.7, 5.2 Hz, 1H), 2.72 - 2.58 (m, 3H), 1.87 (ddd, *J* = 21.3, 13.0, 3.3 Hz, 2H), 1.35 - 1.21 (m, 13H), 1.14 (q, *J* = 11.8 Hz, 2H). MS m/z: 510.45 (M+H).

Example 12:

(S)-N-(1-([1,1'-biphenyl]-4-yl)-3-((2,2,6,6-tetramethylpiperidin-4-yl) amino) propan -2-yl)-2-chloronicotinamide (Cpd012)

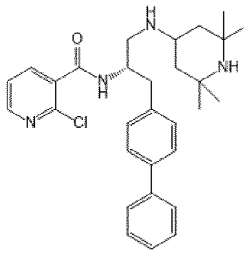

$^1$H NMR (400 MHz, DMSO-$d_6$) δ 8.52 - 8.42 (m, 2H), 7.70 (dd, *J* = 7.6, 2.0 Hz, 1H), 7.66 (dd, *J* = 7.2, 1.8 Hz, 2H), 7.63 - 7.58 (m, 2H), 7.52 - 7.42 (m, 3H), 7.36 (d, *J* = 7.8 Hz, 3H), 4.26 - 4.16 (m, 1H), 2.98 (dd, *J* = 13.7, 5.5 Hz, 2H), 2.90 (s, 0H), 2.77 (dd, *J* = 13.7, 8.4 Hz, 1H), 2.73 (s, 1H), 2.72 (s, 1H), 1.85 (s, 2H), 1.30 - 1.21 (m, 14H), 1.06 (s, 3H). MS m/z: 505.3 (M+H).

Example 13:

(S)-2-chloro-N-(1-(4-((2,6-dichlorobenzyl) oxy) phenyl)-3-((2,2,6,6-tetramethylpiperidin-4-yl) amino) propan -2-yl) nicotinamide (Cpd013)

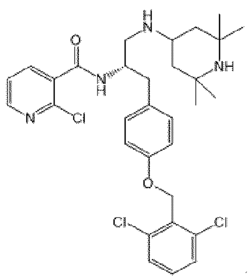

$^1$H NMR (400 MHz, DMSO-$d_6$) δ 8.49 - 8.40 (m, 2H), 8.32 (s, 1H), 7.69 (dd, *J* = 7.5, 2.0 Hz, 1H), 7.57 (d, *J* = 8.0 Hz, 2H), 7.48 (ddd, *J* = 9.0, 6.0, 4.0 Hz, 2H), 7.21 (d, *J* = 8.2 Hz, 2H), 7.00 (d, *J* = 8.5 Hz, 2H), 5.20 (s, 2H), 4.15 (d, *J* = 7.7 Hz, 1H), 3.30 (s, 1H), 3.01 (s, 1H), 2.93 - 2.83 (m, 1H), 2.69 (dt, *J* = 13.7, 9.1 Hz, 3H), 2.03 - 1.85 (m, 2H), 1.35 (dd, *J* = 7.9, 2.7 Hz, 11H), 1.28 - 1.17 (m, 2H), 1.14 (d, *J* = 13.1 Hz, 1H). MS m/z: 605.35 (M+H).

Example 14:

(S)-N-(1-([1,1'-biphenyl]-4-yl)-3-((2,2,6,6-tetramethylpiperidin-4-yl) amino) propan -2-yl) cinnamide (Cpd014)

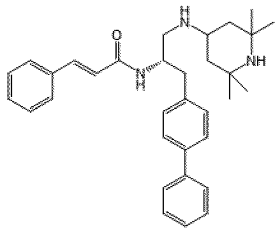

$^1$H NMR (400 MHz, DMSO-$d_6$) δ 8.03 (d, *J* = 8.3 Hz, 1H), 7.79 (s, 4H), 7.69 - 7.61 (m, 2H), 7.56 (dd, *J* = 18.4, 7.5 Hz, 4H), 7.51 - 7.29 (m, 9H), 6.63 (d, *J* = 15.8 Hz, 1H), 4.14 (dt, *J* = 8.1, 5.3 Hz, 1H), 2.96 (dd, *J* = 13.9, 5.8 Hz, 2H), 2.76 (dd, *J* = 13.8, 8.0 Hz, 1H), 2.68 (d, *J* = 6.2 Hz, 2H), 2.58 (s, 0H), 1.88 (t, *J* = 11.2 Hz, 2H), 1.33 - 1.21 (m, 13H), 1.12 (d, *J* = 11.3 Hz, 2H). MS m/z: 496.35 (M+H).

Example 15:

(S)-N-(1-(4-((2,6-Dichlorobenzyl) oxy) phenyl)-3-((2,2,6,6-tetramethylpiperidin-4-yl) amino) propan -2-yl) cinnamide (Cpd015)

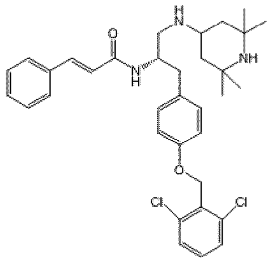

$^1$H NMR (400 MHz, DMSO-$d_6$) δ 7.97 (d, *J* = 8.4 Hz, 1H), 7.62 - 7.51 (m, 4H), 7.50 - 7.34 (m, 5H), 7.22 - 7.15 (m, 2H), 7.01 - 6.94 (m, 2H), 6.62 (d, *J* = 15.8 Hz, 1H), 5.18 (s, 2H), 4.08 (q, *J* = 6.7 Hz, 1H), 2.97 (s, 1H), 2.85 (dd, *J* = 13.8, 5.7 Hz, 1H), 2.67 (dd, *J* = 13.5, 7.4 Hz, 3H), 1.97 - 1.84 (m, 2H), 1.33 (d, *J* = 7.9 Hz, 11H), 1.24 (d, *J* = 3.4 Hz, 1H), 1.16 (q, *J* = 11.7 Hz, 2H). MS m/z: 594.25 (M+H).

Example 16:

(S)-N-(1-([1,1'-biphenyl]-4-yl)-3-((2,2,6,6-tetramethylpiperidin-4-yl) amino) propan -2-yl)-2,2-bis (4-chlorophenoxy) acetamide (Cpd016)

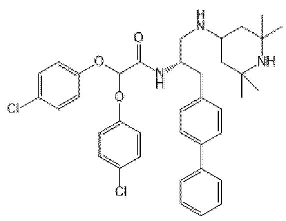

$^1$H NMR (400 MHz, DMSO-$d_6$) δ 8.41 (d, *J* = 8.7 Hz, 1H), 7.64 (d, *J* = 7.6 Hz, 2H), 7.56 (d, *J* = 7.9 Hz, 2H), 7.46 (t, *J* = 7.6 Hz, 2H), 7.39 - 7.25 (m, 8H), 7.04 - 6.92 (m, 5H), 6.17 (s, 1H), 4.09 (d, *J* = 8.3 Hz, 1H), 2.98 - 2.89 (m, 2H), 2.77 (dd, *J* = 13.8, 8.9 Hz, 1H), 2.68 (d, *J* = 6.4 Hz, 2H), 1.87 (d, *J* = 13.3 Hz, 1H), 1.77 (d, *J* = 13.8 Hz, 1H), 1.61 (s, 1H), 1.32 (d, *J* = 6.6 Hz, 6H), 1.30 (s, 6H), 1.25 (d, *J* = 8.7 Hz, 1H), 1.13 (q, *J* = 11.3, 10.8 Hz, 2H). MS m/z: 660.15 (M+H).

Example 17:

(S)-2,2-bis (4-chlorophenoxy)-N-(1-(4-((2,6-dichlorobenzyl) oxy) phenyl)-3-((2,2,6,6-tetramethylpiperidin-4-yl) amino) propan -2-yl) acetamide (Cpd017)

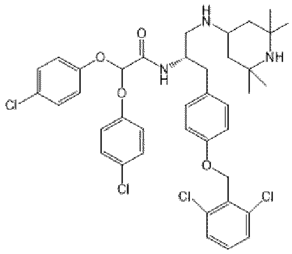

$^1H$ NMR (400 MHz, DMSO-$d_6$) δ 8.33 (d, *J* = 8.7 Hz, 1H), 7.56 (d, *J* = 8.0 Hz, 2H), 7.51 - 7.43 (m, 1H), 7.35 (t, *J* = 9.1 Hz, 4H), 7.16 (d, *J* = 8.2 Hz, 2H), 7.05 - 6.93 (m, 7H), 6.16 (s, 1H), 5.20 (s, 2H), 4.01 (q, *J* = 7.3 Hz, 1H), 2.91 (s, 2H), 2.83 (dd, *J* = 13.7, 5.1 Hz, 1H), 2.67 (dd, *J* = 23.6, 7.6 Hz, 3H), 1.85 (d, *J* = 13.4 Hz, 1H), 1.76 (d, *J* = 13.0 Hz, 1H), 1.35 - 1.27 (m, 12H), 1.24 (s, 1H), 1.21 - 1.03 (m, 2H). MS m/z: 760.35 (M+H).

II. Biological activity test of the compounds of the present application

Example 18: in vitro anti-tumor activity assay of the example compounds

Five human tumor cell lines were selected and cultured in RPMI-1640 (Gibco) or DMEM (Gibco) or GMEM/F12 medium (Gibco) containing 10% inactivated fetal bovine serum, 100 U/ml penicillin and 100 U/ml streptomycin in an incubator containing 5% $CO_2$ at 37°C. Logarithmically growing cells were seeded in 96-well plates (100 μl per well, 2 x $10^4$ cells) using the MTT method, while setting blank control wells. After overnight incubation in the incubator, 100 μl of drug (final concentration 25 μM diluted to 0.39 μM, a total of 7 groups of drug concentrations) was added, an equal volume of culture medium was added in the blank control wells, and 3 duplicate wells were set for each drug concentration. After 48 h of culture, the supernatant was discarded. After washing twice with PBS, 100 μl of MTT (5mg/ml) was added to each well, and the culture was continued for 2 h. Absorbance ($A_{492nm}$) was measured by a microplate reader to calculate the inhibition rate of the drug on tumor cells.

$IC_{50}$ values of compounds on tumor cells were calculated using GraphPad software. The in vitro activity results of the compounds of the three examples are shown in Table 2, and the inhibition rates against different cancer cells are comparable to those of PL-AC-15 and PL-AC-202, which are superior to Cisplatinum.

Table 2. In vitro anti-tumor activity $IC_{50}$ (μM) of example compounds

| Tumor cell | Culture medium | Cpd001 | Cpd009 | Cpd014 | PL-AC-202 | PL-AC-15 | Cisplati num |
|---|---|---|---|---|---|---|---|
| Prostate cancer PC-3 | F12 | 3.03 | 3.73 | 3.31 | 1.65 | 4.31 | 68.60 |
| Prostate cancer LNcap | RPMI-1640 | 3.76 | 1.51 | 1.02 | 3.24 | 1.92 | 11.56 |
| Liver cancer HepG2 | High-sugar DMEM | 0.97 | 1.47 | 1.09 | 0.43 | 1.91 | 30.85 |
| Lung cancer A549 | F12 | 2.69 | 1.94 | 2.34 | 3.05 | 4.38 | 33.95 |
| Lung cancer H460 | High-sugar DMEM | 0.99 | 2.26 | 1.58 | 1.65 | 2.37 | 20.38 |

Example 19: in vivo anti-tumor efficacy assay of the example compound (Cpd014)

An in vivo efficacy evaluation model was established in BALB/c nude mice. Each mouse was inoculated subcutaneously with 0.15 mL of tumor cell suspension (A549 lung cancer cells, 2 x $10^7$ cells/ml) in the axillary fossa of the right forelimb. When the inoculated cells grew to 300 $mm^3$, the administration was started. On the $22^{nd}$ day after inoculation, the mice were intraperitoneally administered (20 mg/kg) once per two days for a total of 10 times. On the 2nd day after the last administration, all the tumor-bearing mice were sacrificed, the tumors were weighed and the tumor inhibition rate was calculated. The results showed that the tumor inhibition rate of Cpd014 was higher than that of PL-AC-202 and docetaxel.

Table 3 Results of in vivo anti-tumor activity of the example compounds

| Group | Mean tumor weight (g) (mean ± SD) | Tumor inhibition rate |
|---|---|---|
| Model | 0.666 ±0.315 | / |
| Cpd014 | 0.448 ±0.128 | 32.73% |
| PL-AC-202 | 0.537 ±0.198 | 19.37% |
| Docetaxol | 0.631 ±0.231 | 5.29% |

While the present application has been particularly shown and described with reference to preferred embodiments thereof, it will be understood by those skilled in the art that various changes in form and details may be made therein. It is intended that the present application be construed as including all such modifications and alterations insofar as they come within the scope of the appended claims or the equivalents thereof.

## Industrial application

The tripeptides of Formula I provided by the present application are novel compounds that have been designed de novo. The compounds of the present application are bradykinin receptor antagonists that inhibit the growth and invasion of tumor cells by inhibiting the binding of bradykinin to its receptor and further inhibiting the development of tumors. Compounds of similar structure may have the same mechanism of action, and understanding the mechanism of action of different compounds will help to have a full understanding of the clinical application prospect and possible problems of the compounds and their analogues in the present application, and make research and development more targeted.

## Claims

**1.** A compound with a structural formula represented by Formula I, or a pharmaceutically acceptable salt, ester, solvate, or isomer thereof:

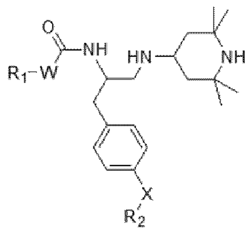

Formula I

wherein the $R_1$ is selected from any one of the following groups: $C_{1-10}$ alkyl, $C_{2-8}$ alkenyl, $C_{2-8}$ alkynyl, $C_{3-12}$ cycloalkyl, 3-12 membered heterocyclyl, $C_{6-14}$ aryl, oxy $C_{6-14}$ aryl, or oxy $C_{5-14}$ arheteryl, nitrogen-based $C_{6-14}$ aryl, or nitrogen-based $C_{5-14}$ arheteryl, 5-14 membered heteroaryl;

the hydrogen on $R_1$ is optionally substituted by one or more of the following substituents: halogen, $C_{1-10}$ alkyl, $C_{2-8}$ alkenyl, $C_{2-8}$ alkynyl, $C_{3-12}$ cycloalkyl, 3-12 membered heterocyclyl, $C_{6-14}$ aryl, oxy $C_{6-14}$ aryl or oxy $C_{5-14}$ arylheteryl, nitrogen-based $C_{6-14}$ aryl or nitrogen-based $C_{5-14}$ arylheteryl, 5-14 membered heteroaryl, -CN, $-NO_2$, $-CF_2H$, $-CF_2OH$, $-CF_3$, $-OCF_3$, $-CR^1R^2R^3$, $-OR^1$, $-O(C=O)R^1$, $-O(C=O)OR^1$, $-O(C=O)NR^2R^3$, $-(C=O)R^1$, $-(C=O)OR^1$, $-(C=O)NR^2R^3$, $-SR^1$, $-(S=O)_mR^1$, $-NR^2R^3$, $-NR^4(C=O)R^1$, $-NR^4C(=O)NR^2R^3$, $-NR^4C(=O)OR^1$, $-NR^4S(=O)_mNR^2R^3$, $-NR^4S(=O)_mOR^1$ or $-NR^4S(=O)_mR^1$, or groups of adjacent atoms on $R_1$ combine to form $C_{3-12}$ cycloalkyl, $C_{6-12}$ aryl, 3-12 membered heterocyclic and 5-12 membered heteroaryl ring groups;

wherein $R^1$, $R^2$, $R^3$, and $R^4$ are independently selected from hydrogen, halogen or any group selected from the group consisting of: $C_{1-10}$ alkyl, $C_{2-8}$ alkenyl, $C_{2-8}$ alkynyl, $C_{3-12}$ cycloalkyl, 3-12 membered heterocyclyl, $C_{6-14}$ aryl, oxy $C_{6-14}$ aryl, or oxy $C_{5-14}$ arheteryl, nitrogen-based $C_{6-14}$ aryl, or nitrogen-based $C_{5-14}$ arheteryl, 5-14 membered heteroaryl, or any two of $R^1$, $R^2$, $R^3$, and $R^4$ bound to the same nitrogen atom combine with the nitrogen to which they are bound to form a 3-12 membered heterocyclyl or 5-12 membered heteroaryl, optionally containing 1 to 3 additional heteroatoms selected from N, O and S, or any two of $R^1$, $R^2$, and $R^3$ bound to the same carbon atom combine to form a $C_{3-12}$ cycloalkyl, $C_{6-12}$ aryl, 3-12 membered heterocyclyl, or 5-12 membered heteroaryl; and each hydrogen in $R^1$, $R^2$, $R^3$, and $R^4$ is optionally substituted with $R^5$, or two hydrogen

atoms on the same carbon atom in $R^1$, $R^2$, $R^3$, and $R^4$ are optionally oxo substituents;

$R^5$ is independently selected from the group consisting of: hydrogen, halogen, $C_{1-10}$ alkyl, $C_{2-8}$ alkenyl, $C_{2-8}$ alkynyl, $C_{3-12}$ cycloalkyl, 3-12 membered heterocyclyl, $C_{6-14}$ aryl, oxy $C_{6-14}$ aryl, or oxy $C_{5-14}$ arheteryl, nitrogen-based $C_{6-14}$ aryl, or nitrogen-based $C_{5-14}$ arheteryl, 5-14 membered heteroaryl, -CN, $-NO_2$, -OH, $-NH_2$, partially or fully halogenated $C_{1-5}$ alkyl, $-C(=O)(CH_2)_nCH_3$, $-C(=O)O(CH_2)_nCH_3$, -C(=O)OH, $-C(=O)N[(CH_2)_nCH_3]_2$, $-C(=O)NH_2$, $-C(=O)NH(CH_2)_nCH_3$, $-NH(CH_2)_nCH_3$, $-N[(CH_2)_nCH_3]_2$, $-N(CH_2)_nCH_3C(=O)(CH_2)_nCH_3$, $-N(CH_2)_nCH_3C(=O)NH(CH_2)_nCH_3$, $-N(CH_2)_nCH_3C(=O)N[(CH_2)_nCH_3]_2$, $-N(CH_2)_nCH_3C(=O)NH_2$, $-N(CH_2)_nCH_3C(=O)O(CH_2)_nCH_3$, $-N(CH_2)_nCH_3C(=O)OH$, $-NHC(=O)(CH_2)_nCH_3$, $-NHC(=O)NH(CH_2)_nCH_3$, $-NHC(=O)N[(CH_2)_nCH_3]_2$, $-NHC(=O)NH_2$, $-NHC(=O)O(CH_2)_nCH_3$, -NHC(=O)OH, $-N(CH_2)_nCH_3S(=O)_m(CH_2)_nCH_3$, $-NHS(=O)_m(CH_2)_nCH_3$, $-O(CH_2)_nCH_3$, =O, $-OC(=O)(CH_2)_nCH_3$, $OC(=O)O(CH_2)_nCH_3$, $-OC(=O)N[(CH_2)_nCH_3]_2$, $-OC(=O)NH(CH_2)_nCH_3$, $-OC(=O)NH_2$, $-S(=O)_m(CH2)_nCH_3$, $-OS(=O)_m(CH2)_nCH_3$, $-S(=O)_mNH(CH_2)_nCH_3$, $-S(=O)_mN[(CH_2)_nCH_3]_2$;

the m is selected from 1 or 2, and

the n is selected from 1, 2, 3, 4, or 5;

$R_2$ is selected from any one of the following: H, halogen, $C_{1-10}$ alkyl, oxy $C_{1-8}$ alkyl, $C_{2-8}$ alkenyl, $C_{2-8}$ alkynyl, $C_{3-12}$ cycloalkyl, 3-12 membered heterocyclyl, $C_{6-14}$ aryl, oxy $C_{6-14}$ aryl, oxy $C_{5-14}$ arylheteryl, nitrogen-based $C_{6-14}$ aryl, nitrogen-based $C_{5-14}$ arylheteryl, 5-14 membered heteroaryl; the hydrogen on $R_2$ is optionally substituted by one or more of the following substituents: halogen, -CN, $-NO_2$, $-CF_2H$, $-CF_2OH$, $-CF_3$, $-OCF_3$, $-CR^6R^7R^8$, $-OR^6$, $-O(C=O)R^6$, $-O(C=O)OR^6$, $-O(C=O)NR^7R^8$, $-(C=O)R^6$, $-(C=O)OR^6$, $-(C=O)NR^7R^8$, $-SR^6$, $-(S=O)R^6$, $-S(=O)_2R^6$, $-NR^7R^8$, $-NR^9(C=O)R^6$, $-NR^9C(=O)NR^7R^8$, $-NR^9C(=O)OR^6$, $-NR^9S(=O)_mNR^7R^8$, $-NR^9S(=O)_mOR^6$ or $-NR^9S(=O)_mR^6$, or groups of adjacent atoms on $R_2$ combine to form $C_{3-12}$ cycloalkyl, $C_{6-12}$ aryl, 3-12 membered heterocyclic, and 5-12 membered heteroaromatic ring;

wherein $R^6$, $R^7$, $R^8$, and $R^9$ are independently selected from hydrogen or any group selected from the group consisting of: $C_{1-10}$ alkyl, $C_{2-8}$ alkenyl, $C_{2-8}$ alkynyl, $C_{3-12}$ cycloalkyl, 3-12 membered heterocyclyl, $C_{6-14}$ aryl, oxy $C_{6-14}$ aryl, or oxy $C_{5-14}$ arheteryl, nitrogen-based $C_{6-14}$ aryl, or nitrogen-based $C_{5-14}$ arheteryl, 5-14 membered heteroaryl, or any two of R1, R2, R3, and R4 bound to the same nitrogen atom combine with the nitrogen to which they are bound to form a 3-12 membered heterocyclic or 5-12 membered heteroaryl, optionally containing 1 to 3 additional heteroatoms selected from N, O, and S; or any two of $R^6$, $R^7$, $R^8$, and $R^9$ bound to the same carbon atom combine to form a $C_{3-12}$ cycloalkyl, $C_{6-12}$ aryl, 3-12 membered heterocyclyl, or 5-12 membered heteroaryl; and each hydrogen in $R^6$, $R^7$, and $R^8$ is optionally substituted with $R^{10}$, or two hydrogen atoms on the same carbon atom in $R^6$, $R^7$, $R^8$, and $R^9$ are optionally oxo substituents;

$R^{10}$ is independently selected from: hydrogen, halogen, $C_{1-10}$ alkyl, $C_{2-8}$ alkenyl, $C_{2-8}$ alkynyl, $C_{3-12}$ cycloalkyl, 3-12 membered heterocyclyl, $C_{6-14}$ aryl, oxy $C_{6-14}$ aryl or oxy $C_{5-14}$ arylheteryl, nitrogen-based $C_{6-14}$ aryl or nitrogen-based $C_{5-14}$ arylheteryl, 5-14 membered heteroaryl, -CN, $-NO_2$, -OH, $-NH_2$, partially or fully halogenated $C_{1-5}$ alkyl, $-C(=O)(CH_2)_nCH_3$, $-C(=O)O(CH_2)_nCH_3$, -C(=O)OH, $-C(=O)N[(CH_2)_nCH_3]_2$, $-C(=O)NH_2$, $-C(=O)NH(CH_2)_nCH_3$, $-NH(CH_2)_nCH_3$, $-N[(CH_2)_nCH_3]_2$, $-N(CH_2)_nCH_3C(=O)(CH_2)_nCH_3$, $-N(CH_2)_nCH_3C(=O)NH(CH_2)_nCH_3$, $-N(CH_2)_nCH_3C(=O)N[(CH_2)_nCH_3]_2$, $-N(CH_2)_nCH_3C(=O)NH_2$, $-N(CH_2)_nCH_3C(=O)O(CH_2)_nCH_3$, $-N(CH_2)_nCH_3C(=O)OH$, $-NHC(=O)(CH_2)_nCH_3$, $-NHC(=O)NH(CH_2)_nCH_3$, $-NHC(=O)N[(CH_2)_nCH_3]_2$, $-NHC(=O)NH_2$, $-NHC(=O)O(CH_2)_nCH_3$, -NHC(=O)OH, $-N(CH_2)_nCH_3S(=O)_m(CH_2)_nCH_3$, $-NHS(=O)_m(CH_2)_nCH_3$, $-O(CH_2)_nCH_3$, =O, $-OC(=O)(CH_2)_nCH_3$, $OC(=O)O(CH_2)_nCH_3$, $-OC(=O)N[(CH_2)_nCH_3]_2$, $-OC(=O)NH(CH_2)_nCH_3$, $-OC(=O)NH_2$, $-S(=O)_m(CH2)_nCH_3$, $-OS(=O)_m(CH2)_nCH_3$, $-S(=O)_mNH(CH_2)_nCH_3$, $-S(=O)_mN[(CH_2)_nCH_3]_2$;

the m is selected from 1 or 2;

the n is selected from 1, 2, 3, 4, or 5;

W represents a bond or is selected from any one of the following groups: $C_{1-8}$ alkylene, $C_{2-8}$ alkenylene, $C_{2-8}$ alkynylene, $C_{3-8}$ cycloalkylene, 3-8 membered heterocyclylene, oxy $C_{1-8}$ alkylene, -O-, -NH-, amino $C_{1-8}$ alkylene, or any of the above groups wherein one or more of the hydrogens are substituted by halogen, and any of the above groups wherein the hydrogens are substituted by $R^{11}$, wherein $R^{11}$ is as defined for $R^1$;

X represents a chemical bond or is selected from any one of the following groups: $C_{1-8}$ alkylene, -O-, oxy $C_{1-8}$ alkylene, -NH-, amino $C_{1-8}$ alkylene, mercapto $C_{1-8}$ alkylene, oxymercapto $C_{1-8}$ alkylene, -S(=O)NH-, $-S(=O)_2NH-$, or any of the above groups in which one or more hydrogens on X are substituted by halogen, and any of the above groups in which a hydrogen on X is substituted by $R^{12}$, wherein $R^{12}$ is as defined for $R^1$.

**2.** The compound, or a pharmaceutically acceptable salt, ester, solvate, or isomer thereof according to claim 1, wherein:

the $R_1$ is selected from any one of the following groups: $C_{1-4}$ alkyl, $C_{2-4}$ alkenyl, $C_{3-7}$ cycloalkyl, phenyl, phenoxy, fluorenyl, pyridyl, substituted pyridyl, and a group in which hydrogen of $R_1$ is optionally substituted with $R^{13}$, wherein $R^{13}$ is as defined for $R^{10}$ in claim 1; $R_2$ is selected from any one of the following: H, halogen, phenyl, biphenyl, 5-6 membered heteroaryl, $C_{3-8}$ cycloalkyl, 3-8 membered heterocyclyl, and a group wherein any

hydrogen on $R_2$ is substituted with $R^{14}$, wherein $R^{14}$ is as defined for $R^{10}$ in claim 1:

W represents a chemical bond or is selected from any one of the following groups: $C_{1-4}$ alkylene, $C_{2-6}$ alkenylene, $-OCH_2-$, $-CH_2O-$;

X represents a chemical bond or is selected from any one of the following groups: $-CH_2-$, $-CH_2CH_2-$, oxy $C_{1-4}$ alkylene.

**3.** The compound, or a pharmaceutically acceptable salt, ester, solvate, or isomer thereof according to claim 2, wherein:

$R_1$ in Formula I in combination with the W group comprises, but is not limited to, the following moieties:

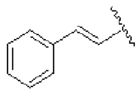 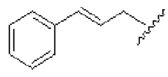 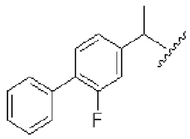 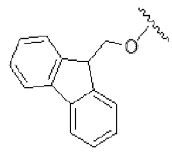

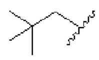 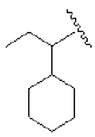 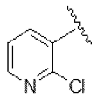 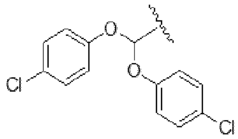

X in the Formula I in combination with $R_2$ comprises, but is not limited to, the following moieties:

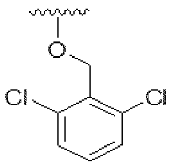 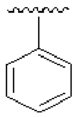 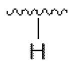 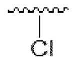

**4.** The compound, or a pharmaceutically acceptable salt, ester, solvate, or isomer thereof according to any one of claims 1 to 3, wherein: the compound of Formula I is selected from any one of the following compounds:

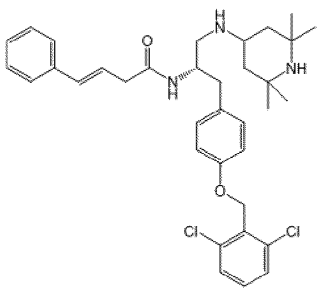 ; 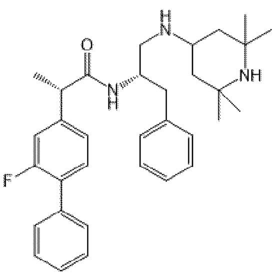 ; 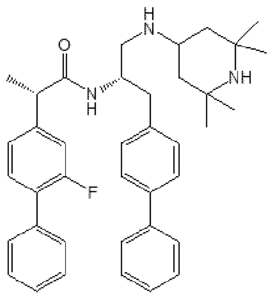 ;

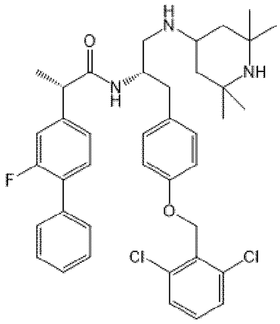 ; 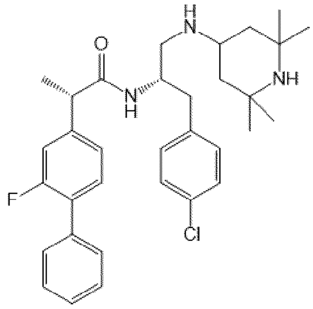 ; 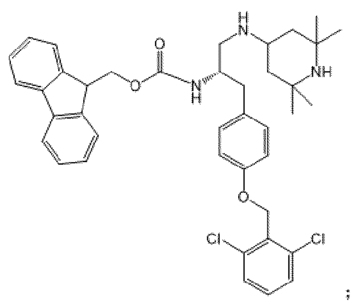 ;

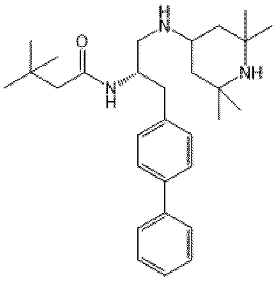

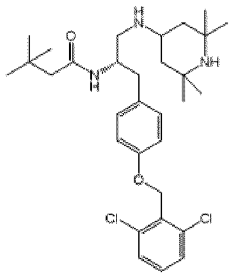
;

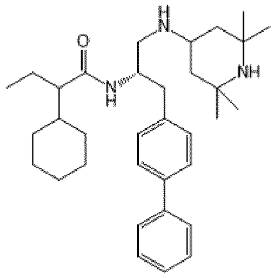
;

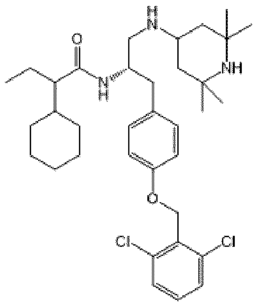
;

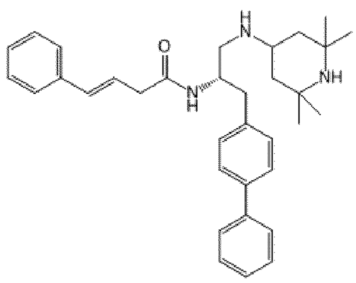
;

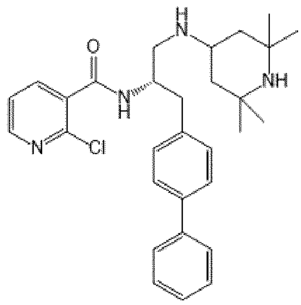
;

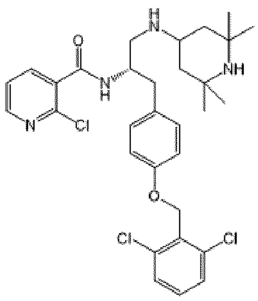
;

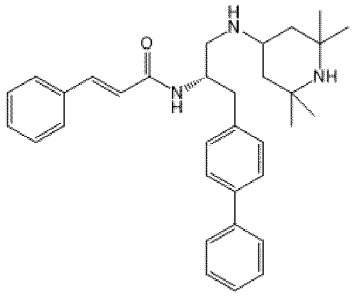
;

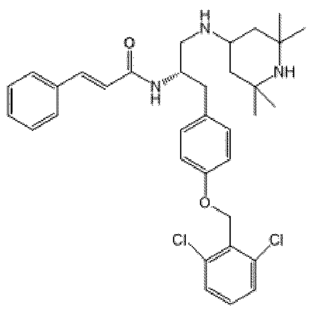
;

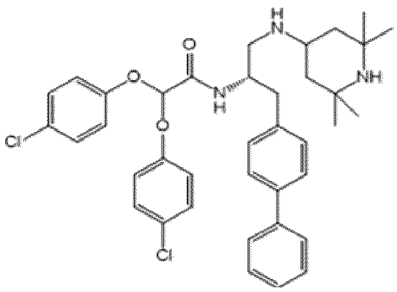
;

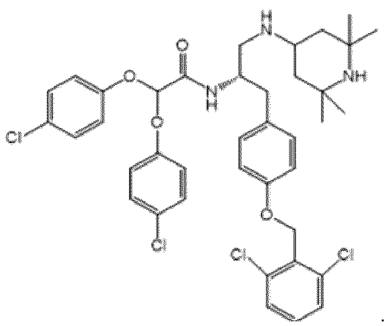
.

**5.** A preparation method for the compound of Formula I according to any one of claims 1 to 4, comprising steps of:

activating $R_1$-W-COOH with oxalyl chloride and reacting with Intermediate 1 or Intermediate 2 at room temperature to obtain the corresponding final product of Formula I;

$R_1$, and W in the $R_1$-W-COOH are defined as in Formula I in claim 1;

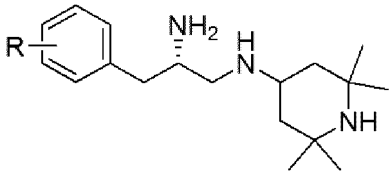

Intermediate 1

R in the Intermediate 1 is -X-$R_2$, wherein X is defined as in Formula I, except that X is -$OCH_2$-, and $R_2$ in the Intermediate 1 is as defined in Formula I;

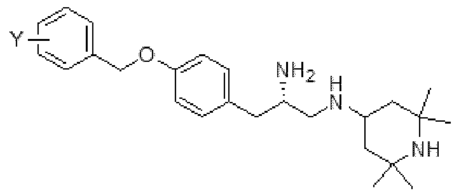

Intermediate 2

in the Intermediate 2, Y represents a group wherein the hydrogen in the phenyl ring to which Y is attached is substituted by one or more $R^{15}$, wherein $R^{15}$ is as defined for $R^1$ in Formula I in claim 1.

**6.** The preparation method according to claim 5, wherein: a synthetic route of Intermediate 1 is shown below:

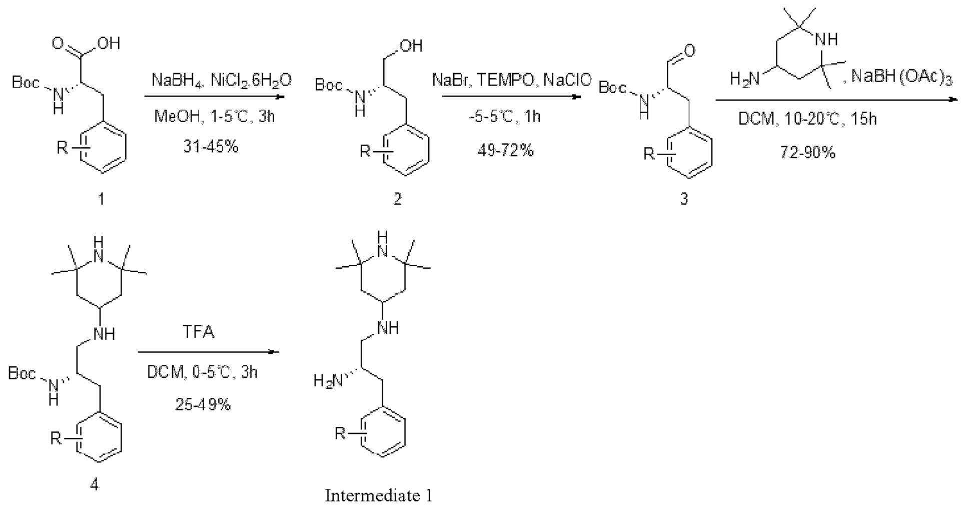

1 2 3 4

Intermediate 1

the specific preparation method is as follows: reducing carboxylic acid 1 under the conditions of $NaBH_4$ to obtain compound 2; oxidizing compound 2 in the conditions of NaClO and TEMPO to obtain compound 3; then reducing the amino group with 1,1,2,2-4methyl-4-aminopiperidine under the condition of $NaBH_4$ to obtain compound 4; and deprotecting compound 4 by TFA in DCM to obtain Intermediate 1;
wherein R in compound 1-4 is as defined in Intermediate 1;
a synthetic route of the Intermediate 2 is shown below:

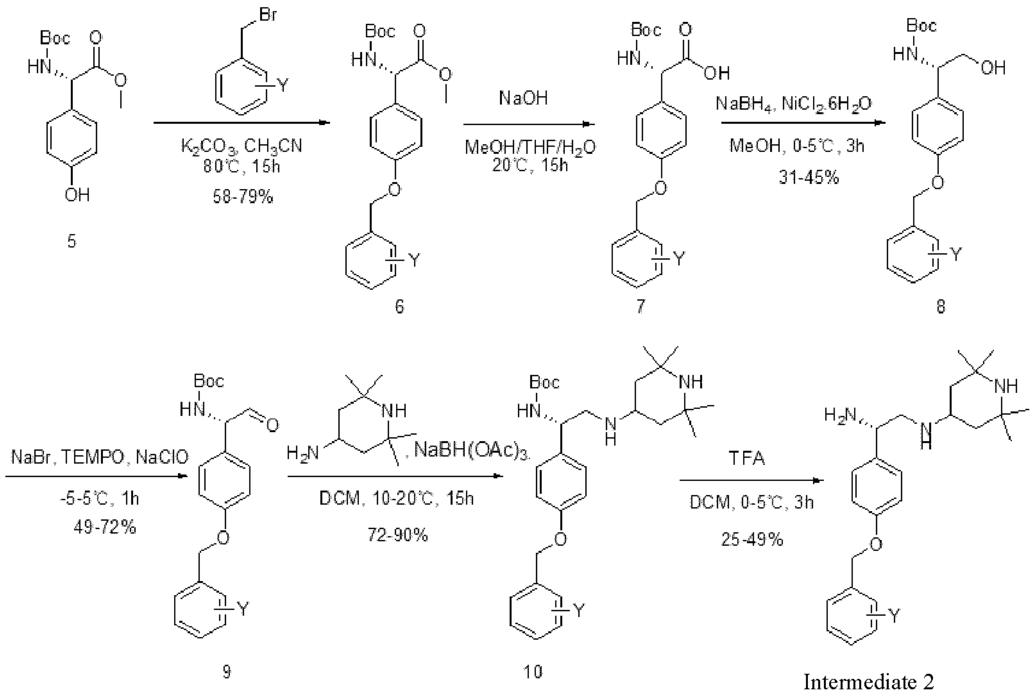


5 6 7 8 9 10 Intermediate 2

a specific preparation method for the Intermediate 2 is as follows: performing a substitution reaction of compound 5 and 2, 6 dichloro-benzyl bromide under the condition of $K_2CO_3$ to obtain compound 6; performing a hydrolysis reaction of compound 6 with NaOH under the condition of MeOH/THF/$H_2O$ to obtain compound 7; reducing compound 7 under the condition of $NaBH_4$ to obtain compound 8, and then performing an oxidation reaction with TEMPO and NaClO to obtain compound 9; subjecting the compound 9 to a reductive amination reaction with 1,1,2,2-4 methyl -4-aminopiperidine to obtain a compound 10; and deprotecting of the compound 10 using TFA in DCM solvent to obtain Intermediate 2.

**7.** Use of a compound, or a pharmaceutically acceptable salt, ester, solvate, or isomer thereof according to any one of claims 1 to 4 in the manufacture of a drug for the prevention and/or treatment of cancer or in the manufacture of a drug for inhibiting the proliferation of cancer cells.

**8.** The use according to claim 7, wherein: the cancer is solid cancer or non-solid cancer, comprising liver cancer, lung cancer, and prostate cancer;
the cancer cells comprise liver cancer cells, lung cancer cells, and prostate cancer cells.

**9.** A drug or pharmaceutical composition for the prevention and/or treatment of cancer, wherein: the drug or pharmaceutical composition comprises an effective amount of the compound of Formula I, or a pharmaceutically acceptable salt, ester, solvate or isomer thereof according to any one of claims 1 to 4.

**10.** The drug or pharmaceutical composition according to claim 8, wherein: the cancer is a solid cancer or non-solid cancer, comprising liver cancer, lung cancer, and prostate cancer.

**11.** A method of treating cancer, comprising administering to a patient in need thereof a therapeutically effective amount of the compound, or a pharmaceutically acceptable salt, ester, solvate, or isomer thereof according to any one of claims 1 to 4, or a therapeutically effective amount of the drug or pharmaceutical composition according to claim 9 or 10.

**12.** The method according to claim 11, wherein: the cancer is a solid cancer or non-solid cancer, comprising liver cancer, lung cancer, and prostate cancer.

**INTERNATIONAL SEARCH REPORT**

International application No.
**PCT/CN2022/141338**

**A. CLASSIFICATION OF SUBJECT MATTER**

C07D211/58(2006.01)i;C07D401/12(2006.01)i;C07D405/12(2006.01)i;A61K31/4468(2006.01)i;A61K31/4545(2006.01)i; A61K31/453(2006.01)i;A61P35/00(2006.01)i

According to International Patent Classification (IPC) or to both national classification and IPC

**B. FIELDS SEARCHED**

Minimum documentation searched (classification system followed by classification symbols)

IPC: C07D; A61K; A61P

Documentation searched other than minimum documentation to the extent that such documents are included in the fields searched

Electronic data base consulted during the international search (name of data base and, where practicable, search terms used)

CNTXT, DWPI, WPABS, CNKI, 万方, WANFANG, STN-REG, CAP, MARPAT: 江苏普莱医药生物技术有限公司, 陈明侠, 姚文军, 陈育新, 乙二胺, 缓激肽受体拮抗剂, 缓激肽, BK, ethylene diamine, STN-structure search

**C. DOCUMENTS CONSIDERED TO BE RELEVANT**

| Category* | Citation of document, with indication, where appropriate, of the relevant passages | Relevant to claim No. |
|---|---|---|
| X | CN 113620862 A (JIANGSU PROTELIGHT PHARMACEUTICAL & BIOTECHNOLOGY CO., LTD.) 09 November 2021 (2021-11-09)<br>see description, embodiment 1, and claims 1-10 | 1-10 |
| A | CN 107382827 A (JIANGSU PROTELIGHT PHARMACEUTICAL & BIOTECHNOLOGY CO., LTD.) 24 November 2017 (2017-11-24)<br>see entire document | 1-10 |
| A | EP 3383865 A1 (BAYER PHARMA AKTIENGESELLSCHAFT) 10 October 2018 (2018-10-10)<br>see entire document | 1-10 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **23 February 2023** | **24 February 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| Facsimile No. **(86-10)62019451** | Telephone No. |

Form PCT/ISA/210 (second sheet) (July 2022)

## INTERNATIONAL SEARCH REPORT

International application No.
**PCT/CN2022/141338**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **11-12**
because they relate to subject matter not required to be searched by this Authority, namely:

Claims 11-12 relate to a diagnosis or treatment method implemented on the human/animal body. Therefore, the subject matter of claims 11-12 falls within subject matter for which a search is not required by the International Searching Authority. (PCT Rule 39.1(iv))

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**INTERNATIONAL SEARCH REPORT**
**Information on patent family members**

International application No.
**PCT/CN2022/141338**

| Patent document cited in search report | | | Publication date (day/month/year) | Patent family member(s) | | | Publication date (day/month/year) |
|---|---|---|---|---|---|---|---|
| CN | 113620862 | A | 09 November 2021 | CN | 113620862 | B | 17 February 2023 |
| CN | 107382827 | A | 24 November 2017 | CN | 107382827 | B | 01 January 2021 |
| EP | 3383865 | A1 | 10 October 2018 | WO | 2017093272 | A1 | 08 June 2017 |
| | | | | EP | 3383865 | B1 | 29 January 2020 |
| | | | | US | 2019218196 | A1 | 18 July 2019 |
| | | | | US | 10654818 | B2 | 19 May 2020 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 107382827 B
- CN 202010386293
- CN 113620862 A